# EUROPÄISCHE PATENTSCHRIFT

(11) **EP 1 888 631 B1**
(45) Veröffentlichungstag und Bekanntmachung des Hinweises auf die Patenterteilung: **23.09.2009**
(21) Anmeldenummer: 06761953.6
(22) Anmeldetag: 31.05.2006
(51) Int. Cl.: C07K 14/51, A61K 39/00

(54) **THERAPEUTSCHE ZUSAMMENSETZUNG ZUR VORBEUGUNG UND BEKÄMPFUNG VON KNOCHENMETASTASEN**
THERAPEUTIC COMPOSITION FOR USE IN THE PREVENTION AND TREATMENT OF BONE METASTASES
COMPOSITION THERAPEUTIQUE POUR PREVENIR ET COMBATTRE LES METASTASES OSSEUSES

(30) Priorität: 31.05.2005 DE 102005024836; 01.02.2006 DE 102006004612
(43) Veröffentlichungstag der Anmeldung: 20.02.2008
(73) Patentinhaber: Jochem, Ralf, 61348 Bad Homburg (DE)
(72) Erfinder: ARMBRUSTER, Franz, Paul, 67240 Bobenheim-Roxheim (DE); BRAUN, Veit, 55122 Mainz (DE); VON EICHEL-STREIBER, Christoph, 55444 Schweppenhausen (DE); JOCHEM, Ralf, 61348 Bad Homburg (DE)
(74) Vertreter: Benedum, Ulrich Max
(86) Internationale Anmeldenummer: PCT/EP2006/005191
(87) Internationale Veröffentlichungsnummer: WO 2006/128689

(56) Entgegenhaltungen:
- WO-A2-02/100899
- WO-A2-02/100901
- BAEUERLE TOBIAS ET AL: "Characterization of a rat model with site-specific bone metastasis induced by MDA-MB-231 breast cancer cells and its application to the effects of an antibody against bone sialoprotein" INTERNATIONAL JOURNAL OF CANCER, Bd. 115, Nr. 2, Juni 2005 (2005-06), Seiten 177-186, XP002406193 ISSN: 0020-7136
- PLUIJM VAN DER G ET AL: "BONE SIALOPROTEIN PEPTIDES ARE POTENT INHIBITORS OF BREAST CANCER CELL ADHESION TO BONE" CANCER RESEARCH, AMERICAN ASSOCIATION FOR CANCER RESEARCH, BALTIMORE, MD, US, Bd. 56, Nr. 8, 1996, Seiten 1948-1955, XP008070268 ISSN: 0008-5472
- BAUERLE TOBIAS ET AL: "TREATMENT OF BONE METASTASIS INDUCED BY MDA-MB-231 BREAST CANCER CELLS WITH AN ANTIBODY AGAINST BONE SIALOPROTEIN" INTERNATIONAL JOURNAL OF ONCOLOGY, EDITORIAL ACADEMY OF THE INTERNATIONAL JOURNAL OF ONCOLOGY,, GR, Bd. 28, Nr. 3, März 2006 (2006-03), Seiten 573-583, XP008069902 ISSN: 1019-6439
- MESNAGE STEPHANE ET AL: "Cell surface-exposed tetanus toxin fragment C produced by recombinant Bacillus anthracis protects against tetanus toxin" INFECTION AND IMMUNITY, Bd. 67, Nr. 9, September 1999 (1999-09), Seiten 4847-4850, XP002433479 ISSN: 0019-9567
- REYMOND C D ET AL: "ANCHORING OF AN IMMUNOGENIC PLASMODIUM FALCIPARUM CIRCUMSPOROZOITE PROTEIN ON THE SURFACE OF DICTYOSTELIUM DISCOIDEUM" JOURNAL OF BIOLOGICAL CHEMISTRY, AMERICAN SOCIETY OF BIOLOCHEMICAL BIOLOGISTS, BIRMINGHAM,, US, Bd. 270, Nr. 21, 26. Mai 1995 (1995-05-26), Seiten 12941-12947, XP002010531 ISSN: 0021-9258
- GEORGIOU G ET AL: "DISPLAY OF HETEROLOGOUS PROTEINS ON THE SURFACE OF MICROORGANISMS: FROM THE SCREENING OF COMBINATORIAL LIBRARIES TO LIVE RECOMBINANT VACCINES" NATURE BIOTECHNOLOGY, NATURE PUBLISHING GROUP, NEW YORK, NY, US, Bd. 15, Januar 1997 (1997-01), Seiten 29-33, XP002951793 ISSN: 1087-0156
- CANO FRANCOIS ET AL: "A surface-displayed cholera toxin B peptide improves antibody responses using food-grade staphylococci for mucosal subunit vaccine delivery" FEMS IMMUNOLOGY AND MEDICAL MICROBIOLOGY, Bd. 25, Nr. 3, 15. August 1999 (1999-08-15), Seiten 289-298, XP002433480 ISSN: 0928-8244
- CORTES-PEREZ NAIMA G ET AL: "Mice immunization with live lactococci displaying a surface anchored HPV-16 E7 oncoprotein." FEMS MICROBIOLOGY LETTERS, Bd. 229, Nr. 1, 5. Dezember 2003 (2003-12-05), Seiten 37-42, XP002433481 ISSN: 0378-1097
- LILJEQVIST SISSELA ET AL: "Surface display of the cholera toxin B subunit on Staphylococcus xylosus and Staphylococcus carnosus" APPLIED AND ENVIRONMENTAL MICROBIOLOGY, Bd. 63, Nr. 7, 1997, Seiten 2481-2488, XP002433482 ISSN: 0099-2240
- GENTSCHEV I ET AL: "The E. coli alpha-hemolysin secretion system and its use in vaccine development" TRENDS IN MICROBIOLOGY, ELSEVIER SCIENCE LTD., KIDLINGTON, GB, Bd. 10, Nr. 1, Januar 2002 (2002-01), Seiten 39-45, XP002247589 ISSN: 0966-842X
- LEENHOUTS KEES ET AL: "Anchoring of proteins to lactic acid bacteria" ANTONIE VAN LEEUWENHOEK, Bd. 76, Nr. 1-4, Juli 1999 (1999-07), Seiten 367-376, XP002433483 ISSN: 0003-6072

## Beschreibung

### GEBIET DER ERFINDUNG

Die Erfindung betrifft Arzneimittel zur Vorbeugung und Behandlung von Knochenmetastasen auf der Basis von Antikörpern gegen nicht-zelluläre Knochenmatrixproteine.

### HINTERGRUND DER ERFINDUNG

Auf eine primäre Krebserkrankung folgen häufig sekundäre Knochentumore. Trotz aller medizinischen Fortschritte sind Knochentumore nicht heilbar, da sie in Regel gestreut auftreten und operativ kaum behandelt werden können. Die Auftretenswahrscheinlichkeit von Knochenmetastasen liegt beim Multiplen Myelom bei größer 96%, beim Mamma- und Prostatakarzinom zwischen 65 und 75%, und bei Karzinomen der Lunge, Niere, Cervix, Blase zwischen 30 und 50%. Systemische Chemotherapien zeigen kaum Wirkung. Erste Erfolge konnten mit therapeutischen Antikörpern erzielt werden. Bei therapeutischen Antikörpern besteht jedoch das Problem, dass sie zumeist gegen Ziele gerichtet sind, die nur auf proliferierenden Tumorzellen vorhanden sind. Im Blutkreislauf zirkulierende nicht-proliferierende Tumorzellen und schlummernde Metastasen werden in der Regel nicht erkannt.

Zwischen dem Auftreten der Knochenmatrixproteine Osteopontin (OPN), Osteonectin (ON) und Bone-Sialoprotein II (BSP) im Primärtumor und späteren Tochtergeschwülsten im Knochen besteht ein enger Zusammenhang. Die Zellen eines osteotroph metastasierenden Primärtumors exprimieren nahezu stets signifikante Mengen BSP oder OPN (Diel IJ et al, Clinic Cancer Res, 1999, 5:3914; Tuck AB et al, J Mammary Gland Biol Neoplasia, 2001, 6:419; Rudland PS et al, Cancer Res 2002, 62:3417; Agrawal D et al, J Natl Cancer Inst, 2002, 94:513; Waltregny D et al, J Bone Miner Res, 2000, 15(5):834; Bellahcéne A et al, J Bone Miner Res, 1996, 11:665; Waltregny D et al, J Nat Cancer Inst, 1998, 90:1000; Bellahcène A et al, Int J Cancer, 1996, 69:350; WO 02 100901 (Immundiagnostik AG), WO 02 25285 A (Smith et al)).

BSP ist ein phosphoryliertes Glycoprotein mit einer relativen Masse von ca. 80 kDa in der SDS-PAGE. Die cDNA für BSP kodiert für eine Peptidsequenz von ca. 33 kDa (Fisher LW et al, J Biol Chem, 1990, 265:2347; US 5 340 934 (Termine)). Es stellt ca. 10 bis 15 % der nicht-kollagenen Proteine in der Knochenmatrix und wird normalerweise von Zellen exprimiert, die an der Bildung von Dentin, Knochen und Knorpel beteiligt sind, beispielsweise von Osteoblasten, sich entwickelnden Osteozyten, hypertrophen Chondrozyten, Odontoblasten und Zementoblasten. Das BSP unterstützt als Adhäsionsmolekül die Anheftung und Ausbreitung von Osteoblasten und Osteoklasten auf der Knochengewebsmatrix. Das Ausschalten des BSP-Gens in Knock-out-Mäusen führte aber zu keiner erkennbaren Störung der Skelettbildung. Dem BSP wird aber eine Beteiligung an der Mikrokalzifikation von Knochen und bei deren Besiedlung durch Tumorzellen zugeschrieben (Castronovo V et al, Int J Oncol, 1998, 12:308, Bellahcène A et al, Int. J. Cancer, 1996, 69:350).

Freies BSP wird in Körperflüssigkeiten vom Komplementfaktor-H mit hoher Affinität gebunden. Es gibt viele Antikörper gegen Peptidstrukturen des BSP, rekombinantes BSP und aus Knochen isoliertes BSP, die alle *nicht* BSP in Serum erkennen und binden (Fisher LW et al, Acta Orthop Scand Suppl. 1995, 266:61; Stubbs JT (III) et al, J Bone Miner Res, 1997, 12(8):1210). Das 150 kDa große Faktor-H-Molekül umschließt sehr wahrscheinlich das BSP-Molekül so, dass diese Antikörper nicht binden können. Dadurch werden Trophoblasten und BSP-produzierenden Tumorzellen auch vor einem Angriff des Immunsystems geschützt (Fedarko NS et al., J. Biol. Chem., 2000, 275, 16666-16672; WO 00/062065). Auch die starke Glykosylierung des BSP wird für diese Beobachtung eine Rolle spielen. Weiterhin ann BSP mittels der RGD-Sequenz (Arginin-Glycin-Aspartat) an alpha(v)beta(3) Integrin-Rezeptoren auf der Zellwand binden. Das BSP ist daher auch beteiligt an der Adhäsion, Ausbreitung und Orientierung der Endothelzellen und die Angiogenese um einen Tumor (Bellahcène A et al, Circ Res. 2000, 86(8):885-91). Diese Eigenschaften machen BSP neben OPN und ON in der Familie der nicht-kollagenen Integrinrezeptor-bindenden Glycoproteine zu einem Ausgangspunkt für Medikamente aller Art (US 5 780 526; US 5 767 071; US 5 792 745; US 5 939 383; US 5 773 412; US 5 849 865).

So gibt es Versuche, die Bindung von BSP an die Vitronectin- beziehungsweise Integrin-Rezeptoren der Tumor- und Epithelzellen durch RGD-Antagonisten zu hemmen (US 6 069 158; US 6 008 213; US 5 849 865; van der Pluijm et al., Cancer Res., 1996, 56, 1948-1955). EP 1 084 719 (DePuy Orthopeadics Inc.) lehrt BSP als Wirkstoff zur Unterstützung der Reparatur von geschädigten Knochen- und Bindegewebe, WO 94 13310 (Alfa-Laval Agriculture Intern. AB) ein BSP-bindendes Protein aus *Staphylococcus aureaus* zur Behandlung von Infektionen und entzündlichen Erkrankungen des Knochens, WO 02/100899 (Armbruster Biotechnology GmbH) einen Wirkstoff gegen Knochenmetastasen auf der Basis von Antikörpern gegen BSP. WO 00/36919 (Univ. Virginia Patent Found.) beschreibt regulatorische Elemente zur Kontrolle und Unterdrückung der BSP-Expression in Tumor- und Bindegewebszellen und EP 0 020 789 (DKFZ) eine Hemmung von Zellmigration und Knochenmetastasenbildung durch Antisense-Oligonucleotide, siehe hierzu ferner Adwan-Hassan et al in Cancer Gene Therapy, 2003:1; Intern J Oncol, 2004, 24:1235-1244; Proc Am Assoc Cancer Res Ann, 2003, 44:56). Die nach dem Prioritätstag veröffentlichte WO 2006/036550 (Trustees of the Univ. of Pennsylvania) beschreibt ferner Vakzine auf der Basis von Listerium und Fusionsproteinen aus Listeriolysin und CD8⁺-T-Zellepitopen (Her-2) zur Behandlung osteotropher Tumore und Carcinome.

Es wird also untersucht, was den Primärtumor, der in der Regel chirurgisch behandelt werden kann, zur Metastasenbildung veranlasst, wie Knochenmetastasen vorgebeugt werden kann und womit sich diese behandeln und gegebenenfalls heilen lassen. Problematisch an den bisherigen Ansätzen ist, dass eine Therapie mit Antisense-Oligonucleotiden oder Antikörpern, sofern möglich, nur über eine begrenzte Zeit wirksam aufrecht erhalten werden kann. Es besteht nicht nur ein Dosis- und Applikationsproblem, sondern es werden auch Autoantikörper gegen die therapeutischen Immunglobuline und regulatorischen Nucleotide entwickelt. Schließlich gibt es auch natürliches endogenes BSP im Körper, so dass eine Immunreaktion gegen körpereigenes BSP durch das Immunsystem blockiert ist. Demgegenüber muss eine Prophylaxe oder die direkte Behandlung von Knochenmetastasen über sehr lange Zeiträume erfolgen, soll sie Aussicht auf Erfolg haben. Die Gefahr des Auftretens von Knochenmetastasen nach einem behandelten osteotrophen Primärtumor besteht über Jahrzehnte, und es besteht daher Bedarf an einer therapeutischen Zusammensetzung, die nachhaltig die Ansiedelung und Bildung von Knochentumoren unterbindet und bestehende Knochenmetastasen bekämpft.

### ZUSAMMENFASSUNG DER ERFINDUNG

Diese Aufgabe wird gelöst durch eine therapeutische Zusammensetzung nach Anspruch 1. Bevorzugte Ausführungsformen der Erfindung sind in den Unteransprüchen beschrieben.

Die therapeutische Zusammensetzung zur Behandlung und Prophylaxe von Knochentumoren und Metastasen, die bevorzugt in Knochengeweben ansiedeln, enthält erfindungsgemäß als Wirkstoff abgetötete oder schwach pathogene Mikroorganismen, die ein Gen für antigene Fragmente des Bonesialoproteins enthalten und ein oder mehrere Bonesialoprotein-Antigene exprimieren, welche sich in mindestens einem Strukturmerkmal von endogenem Bonesialoprotein normaler Osteoblasten unterscheidet, so dass bei einer Verabreichung eine Immunreaktion gegen das veränderte Bonesialoprotein erzeugt wird. Die exprimierten Bonesialoprotein Antigene weisen dabei bevorzugt Strukturmerkmale auf, die spezifisch sind für eine Bonesialoprotein-Isoform, welche von osteotrophen Zellen eines Primärtumors exprimiert wird. Der Mikroorganismus ist erfindungsgemäß ausgewählt aus Bakterien, Viren und Einzellern, bevorzugt aus Gram-positiven Bakterien wie Listerien. Er kann auch ausgewählt werden aus den Spezies Aeromonas, Bartonella, Bruceila, Bacillen, Bacillus subtilis, Lactobacillen, Pseudomonaden, Staphylokokken, Yersinia Campylobacter, Clostridia, Enterobacteriaceae, Legionella, bevorzugt Listeria, besonders bevorzugt Listeria monocytogenes, Mycobacterium, Renibacterium, Rhodococcus, Bakterien der Spezies Yersinia, Escherichia, Shigella, Salmonella, und Bakterien, die in einem eukaryonten Wirtsorganismus lebensfähig sind. Besonders bevorzugt ist, wenn der Mikroorganismus ein oder mehrere antigene Determinanten des Bonesialoproteins auf der Oberfläche verankert trägt, insbesondere tumortypisches Bonesialoprotein oder Fragmente daraus. Der Mikroorganismus kann unterglykosyliertes Bonesialoprotein-Antigen oder Fragmente davon auf der Oberfläche verankert tragen. Für eine hohe therapeutische Wirksamkeit ist wichtig, dass das Bonesialoprotein-Antigen dann ein Epitop enthält, das im Komplex von Komplementfaktor-H und Bonesialoprotein für die Bindung eines Antikörpers frei ist. Erfindungsgemäß enthält ein solches Bonesialoprotein-Antigen ein oder mehrere Kopien folgender Aminosäuresequenzen:

| | |
|---|---|
| YTGLAAIQLPKKAGD | SEQ ID NO. 5 |
| TGLAA | SEQ ID NO. 3 |
| YTGLAA | SEQ ID NO. 4 |
| YESENGEPRGDNYRAYED | SEQ ID NO 6 |
| LKRFPVQGG | SEQ ID NO: 7 |
| EDATPGTGYTGLAAIQLPKKAG | SEQ ID NO: 10 |

In einer Ausführungsform der Erfindung enthält die therapeutische Zusammensetzung als Wirkstoff eine Immunogen mit einem Hapten, das auf Bonesialoprotein aus Tumorzellen vorhanden ist, und bevorzugt die antigene Determinante des Bonesialoproteins in mindestens zwei oder mehr Kopien. Die therapeutische Zusammensetzung gemäß der Erfindung eignet sich zur Behandlung von Tumoren aus der Gruppe mit Prostata-, Mamma-, Lungen-, Nieren- und Schilddrüsentumoren, Tumorerkrankungen des Blutsystems, des Lymphsystems, des Herz-Kreislauf-Systems, des Nervensystems, des Respirationstraktes, des Verdauungstraktes, des endokrinen Systems, der Haut einschließlich Anhangsgebilde, des Bewegungsapparates und des Urogenitaltraktes, einschließlich der Niere.

Die erfindungsgemäße therapeutische Zusammensetzung kann erhalten werden mit einem Verfahren zur Entwicklung einer Wirkstoffzusammensetzung, umfassend die Schritte: (i) Wahl eines krankheitsrelevanten Proteins, zum Beispiel BSP; (ii) Klonierung und Expression einer antigenen Struktur des relevanten Proteins in einem Mikroorganismus, der die antigene Teilstruktur exprimiert, sekretiert und auf der Zelloberfläche, verankert in der Membran, präsentiert; (iii) Hervorrufen von Antikörpern gegen die antigene Teilstruktur des krankheitsrelevanten Proteins; (iv) Prüfen der Antikörper auf eine therapeutische Wirkung. Der Mikroorganismus ist bevorzugt ein Gram-positives Bakterium, besonders bevorzugt Listerium. In dem Verfahren werden dann zum Screenen der Antigene Seren von Säugetieren eingesetzt und diese auf die Anwesenheit von Antikörpern gegen die antigene Struktur des krankheitsrelevanten Proteins untersucht. Zweckmäßigerweise werden in dem Verfahren vor allem krankheitsrelevante Proteine untersucht, die eine physiologische Funktion besitzen bei der Ansiedelung von Tumorzellen im Knochen, beispielsweise die extrazellulären Knochenmatrixproteine Bonesialoprotein (BSP), Osteopontin (OPN), Osteonectin (ON) und Wachstumsfaktoren für osteotrophe Tumore um therapeutisch wirksame Antikörper und Vakzine zu gewinnen.

Die erfindungsgemäße Zusammensetzung ist anwendbar in einem Verfahren zur Behandlung und Prophylaxe von Knochentumoren und Metastasen, die bevorzugt in Knochengeweben ansiedeln, umfassend das Verabreichen von abgetöteten oder schwach pathogenen Mikroorganismen, die ein oder mehrere Bonesialoprotein-Antigene auf der Oberfläche verankert aufweisen, welche sich in mindestens einem Strukturmerkmal von endogenem Bonesialoprotein normaler Osteoblasten unterscheidet, so dass bei Verabreichung eine Immunreaktion erzeugt wird, die gegen den Tumor und dessen streuenden Tumorzellen wirkt. In einer Alternative dieses Verfahrens werden peptidische Moleküle verabreicht oder Trägerproteine mit antigenen Determinanten des Bonesialoproteins, die für tumorgenes BSP charakteristisch sind, so dass bei Verabreichung eine Immunreaktion erzeugt wird, die gegen den Tumor und dessen streuenden Tumorzellen wirkt. Eine andere Alternative dieses Verfahren umfasst das Verabreichen von peptidischen Molekülen oder Trägerproteinen mit antigenen Determinanten des Bonesialoproteins, die für tumorgenes BSP charakteristisch sind, so dass bei Verabreichung eine Immunreaktion erzeugt wird, die gegen den Tumor und dessen streuenden Tumorzellen wirkt. Bevorzugt ist hierbei, wenn die antigenen Determinanten auf den peptidischen Molekülen oder Trägerproteinen mehrfach vorhanden sind oder die peptidischen Molekülen mit beta-Alanin gekoppelt sind.

Erfindungsgemäß enthält die therapeutische Zusammensetzung zur Behandlung und Prophylaxe von Knochentumoren und Metastasen, die bevorzugt in Knochengeweben ansiedeln, als Wirkstoff abgetötete oder schwach pathogene Mikroorganismen, die das Gen für BSP enthalten und zur Expression von ein oder mehreren BSP-Molekülen führen, welche sich in mindestens einem Strukturmerkmal von endogenem BSP normaler Osteoblasten unterscheiden, so dass bei Applikation eine Immunreaktion gegen das veränderte BSP erzeugt wird. Bevorzugt ist dabei, wenn die exprimierten BSP-Moleküle tumorcharakteristische Strukturmerkmale aufweisen, wie sie bei BSP aus osteotrophen Zellen eines Primärtumors zu finden sind. Der zugehörige Mikroorganismus ist ausgewählt aus Bakterien, Viren und Einzeller und ist bevorzugt ein Gram-positives Bakterium, ganz besonders bevorzugt Listerium. Der Mikroorganismus kann aus intrazellulär in Wirtszellen vermehrenden Bakterien ausgewählt sein, beispielsweise aus Bakterien der Spezies Aeromonas, Bartonella, Bruceila, Bacillen, Bacillus subtilis, Lactobacillen, Pseudomonaden, Staphylokokken, Campylobacter, Clostridia, Enterobacteriaceae, Legionella, Listeria, Mycobacterium, Renibacterium, Rhodococcus, Yersinia, Escherichia, Shigella, Salmonella, und insbesondere Bakterien, die in einem eukaryonten Wirtsorganismus lebensfähig sind wie Listeria. Ursprünglich nicht intrazellulär wachsende Bakterien können zudem durch genetische Manipulationen mit Faktoren ausgestattet werden, die den Zutritt zur Zelle erlauben. Vorteilhaft ist, wenn der gentechnisch veränderte Mikroorganismus ein exogenes beziehungsweise heterologes Suizidgen aufweist und eine zielgerichtete, somatischen Transgenität in Wirtzellen herbeiführen kann. Besonders bevorzugt ist die Verwendung von Listerien für die Herstellung der erfindungsmäßen therapeutischen Zusammensetzung.

Ein weiterer Aspekt der Erfindung betrifft eine therapeutische Zusammensetzung, wobei der gentechnisch veränderte Mikroorganismus, zum Beispiel das Listerium, ein BSP-Antigen beziehungsweise eine antigene Determinante des BSP auf der Oberfläche verankert hat, bevorzugt aus humanem BSP und dessen Fragmenten, besonders bevorzugt, aus unterglykosyliertem BSP und dessen Fragmenten. Die exprimierten BSP-Fragmente werden dann im Säugetier als fremd erkannt, insbesondere wenn sie auf der Membran eines Mikroorganismus beziehungsweise auf der Zelloberfläche infizierter Wirtszellen liegen. Die so induzierten eigenen Antikörper binden an das BSP-Antigen der osteotrophen Tumorzellen. Ein analoger Effekt kann vermutlich auch erzielt werden, wenn bakterientypische Aminosäuren wie beta-Alanin (3-Aminopropionsäure) mit den peptidischen Antigendeterminanten des BSP (am C- oder N-terminalen Ende des Peptids oder an beiden Enden) gekoppelt werden. Auch dann wird vermutlich eine immunogene BSPbeziehungsweise Tumor-BSP-Determinate geschaffen, die in einem Anti-BSP-Vakzin genutzt werden kann.

Bevorzugte DNAs zur Herstellung spezifischer Tumor-BSP-Antikörper kodieren unter anderem folgende Sequenzen des humanen Bonesialoproteins (SWISSPROT: SIAL_HUMAN, Acc.No. P21815) und seiner Homologen:
SEQ ID NO: 1
   X-YTGLAAIQLPKKAGD-Z

Das markierte Threonin ist in Tumor-BSP nicht oder unvollständig oder in anderer Form glykosyliert. In einer Ausführungsform wird dieses Threonin in eine nicht glykosylierbare Aminosäure zu überführt. X und Z stehen für Aminosäure- beziehungsweise Peptidreste, z.B. für einen Membrananker, poly(Histidine), Poly(HiS)₅₋₁₂, oder beta-Alanin. SEQ ID NO: 2 kann wie folgt verändert sein: Position 179 Gly→ Val; Position 252 Val → Ala; Position 254 Glu → Asp; Position 279 Asp → Gly.

In einer Ausführungsform der Erfindung führt die therapeutische Zusammensetzung zur Bildung körpereigener Antikörper gegen ein BSP, dessen posttranslationale Glykosylierung im Bereich der Aminosäuren 120 bis 135 (SWISSPROT: SIAL_HUMAN, Acc.No. P21815) gegenüber normalem BSP aus Knochen verändert oder unvollständig ist. Bevorzugt ist die Induktion körpereigener Antikörper, die ein hBSP-Epitop erkennen, welches die Aminosäuresequenz TGLAA (SEQ ID NO: 3) oder YTGLAA (SEQ ID NO: 4) und optional Zuckergruppen sowie ein Trägermolekül umfasst.

Das erfindungsgemäße Vakzin führt also zu körpereigenen Antikörper gegen tumorgenes BSP. Die so induzierte Immunität schützt somit vor einem Andocken von metastasierenden osteotrophen Tumorzellen im Knochengewebe und verhilft zu einer zellvermittelten Zytotoxizität gegen Tumor-BSP-produzierende Zellen.

Ein anderer Aspekt der Erfindung betrifft eine Zusammensetzung, wobei das BSP-Antigen eine antigene Determinante beziehungsweise ein Epitop enthält, das im Komplex von Komplementfaktor-H und BSP für die Bindung eines Antikörpers frei ist. Die antigene Determinante des BSP kann ein oder mehrere Kopien folgender Aminosäuresequenzen enthalten:

| | |
|---|---|
| TGLAA | SEQ ID NO: 3 |
| TGLAA | SEQ ID NO: 4 |
| YTGLAAIQLPKKAGD | SEQ ID NO: 5 |
| YESENGEPRGDNYRAYED | SEQ ID NO: 6 |
| LKRFPVQGG | SEQ ID NO: 7 |

Ein besonderes Ziel ist dabei eine therapeutische Zusammensetzung, dessen Wirkstoff ein Hapten aufweist, welches auf BSP aus Tumorzellen gleichfalls vorhanden ist. Die erfindungsgemäße Zusammensetzung ist geeignet zur Behandlung von Tumoren und Karzinomen aus der Gruppe mit Prostata-, Mamma-, Lungen-, Nieren- und Schilddrüsentumoren, Tumorerkrankungen des Blutsystems, des Lymphsystems, des Herz-Kreislauf-Systems, des Nervensystems, des Respirationstraktes, des Verdauungstraktes, des endokrinen Systems, der Haut einschließlich Anhangsgebilde, des Bewegungsapparates und des Urogenitaltraktes, einschließlich der Niere.

Die erfindungsgemäße therapeutische Zusammensetzung kann erhalten werden mit einem Verfahren zur Entwicklung einer Wirkstoffzusammensetzung beziehungsweise eines Impfstoffs umfassend die Schritte: (i)Wahl eines krankheitsrelevanten Proteins, zum beispiel BSP; (ii) Klonierung und Expression einer antigenen Struktur des relevanten Proteins in einem Mikroorganismus derart, dass er die antigene Struktur exprimiert, sekretiert und auf der Zelloberfläche, verankert in der Membran, präsentiert; (iii) Hervorrufen von Antikörpern gegen die antigene Struktur des krankheitsrelevanten Proteins; (iv) Prüfen der Antikörper auf eine therapeutisch Wirkung und Verwenden des Mikroorganismus in einer therapeutischen Zusammensetzung wie beispielsweise einem Vakzin. Der Mikroorganismus, der die antigene Struktur auf seiner Zelloberfläche präsentiert, ist beispielsweise ein Gram-positives Bakterium, bevorzugt Listerium. Bevorzugte Mikroorganismen bewirken eine somatische Transgenität in Wirtzellen und sie präsentieren die antigene Struktur, einschließlich posttranslationaler Modifikationen, auf der Oberfläche der Wirtszellen. Besonders bevorzugt sind Mikroorganismen wie Listerium, die eine Immuntoleranz gegen einen Antigen-exprimierenden Tumor brechen. Dies kann insbesondere dadurch erreicht werden, dass das Antigen auf der Oberfläche des Mikroorganismus verankert und präsentiert wird oder dass das Antigen mit Bakterien-typischen Aminosäuren wie beta-Alanin gekoppelt wird. Der Mikroorganismus kann vor Schritt (iii) inaktiviert werden. Es liegt nahe, weiterhin Seren von Säugetieren auf die Anwesenheit von Antikörpern gegen die antigene Struktur des krankheitsrelevanten Proteins zu untersuchen, und dabei insbesondere auch Proteine anzuschauen, die eine physiologische Funktion besitzen bei der Ansiedelung von Tumorzellen im Knochen. Diese hierbei interessierenden Proteine sind vor allem die extrazellulären Knochenmatrixproteine Bonesialoprotein (BSP), Osteopontin (OPN), Osteonectin (ON) und die Wachstumsfaktoren für Tumore. Das Verfahren kann als Screening-Verfahren breit eingesetzt werden. Hierbei werden eine Mehrzahl antigener Strukturen von krankheitsrelevanten Proteinen in Mikroorganismen kloniert, exprimiert und auf der Oberfläche verankert und reihenweise Seren von Säugetieren auf Antikörper gegen die antigenen Teilstrukturen getestet, um so therapeutisch wirksame antigene Determinanten, Teilstrukturen und Haptene sowie Antikörper hiergegen heraus zu filtern. Die Anamnese der Säugetieren beziehungsweise der Patienten, insbesondere der mit einer spontaner Heilung, weist dann auch die mögliche Wirksamkeit der Antikörper. Das Ergebnis sind dann therapeutisch wirksame Antikörper und Mikroorganismen mit der antigenen Struktur auf der Oberfläche, und schließlich darauf basierende Vakzine.

Weitere Aspekte und Vorteile der Erfindung sind der detaillierten Beschreibung der Erfindung, den anliegenden Abbildungen und den Beispielen zu entnehmen.

### KURZE BESCHREIBUNG DER ABBILDUNGEN

Es zeigt:

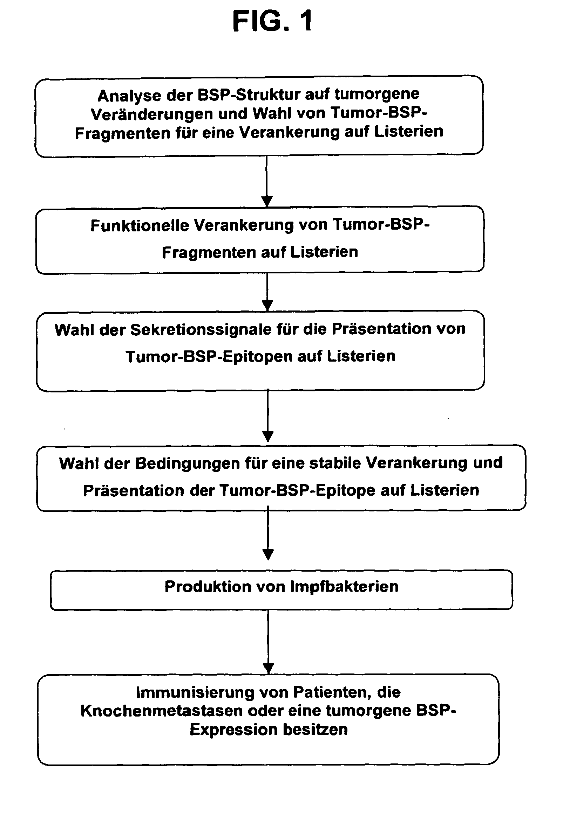

- Fig. 1: ein Blockdiagramm des Verfahrens zur Gewinnung von Bakterien mit antigenen Determinanten aus BSP, welche eine Immunreaktion hervorrufen gegen osteotrophe Tumorzellen und die Ansiedelung von Tumorzellen in Knochen;

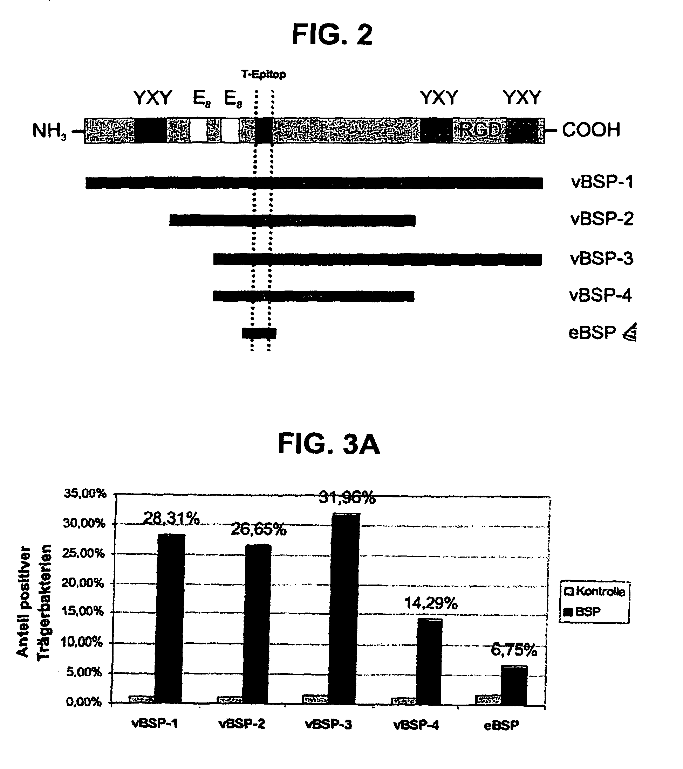

- Fig. 2: eine zeichnerische Darstellung der generierten BSP-Fragmente für die rekombinante Erzeugung von BSP-Antigenabschnitten auf Trägerbakterien; RGD: Zellbindendes Motiv; YXY: Tyrosin-reiche Region; E8: Glutamat-reiche Region; T-Epitop: BSP-Tumor-Epitop (BSP-Position 125 - 130);

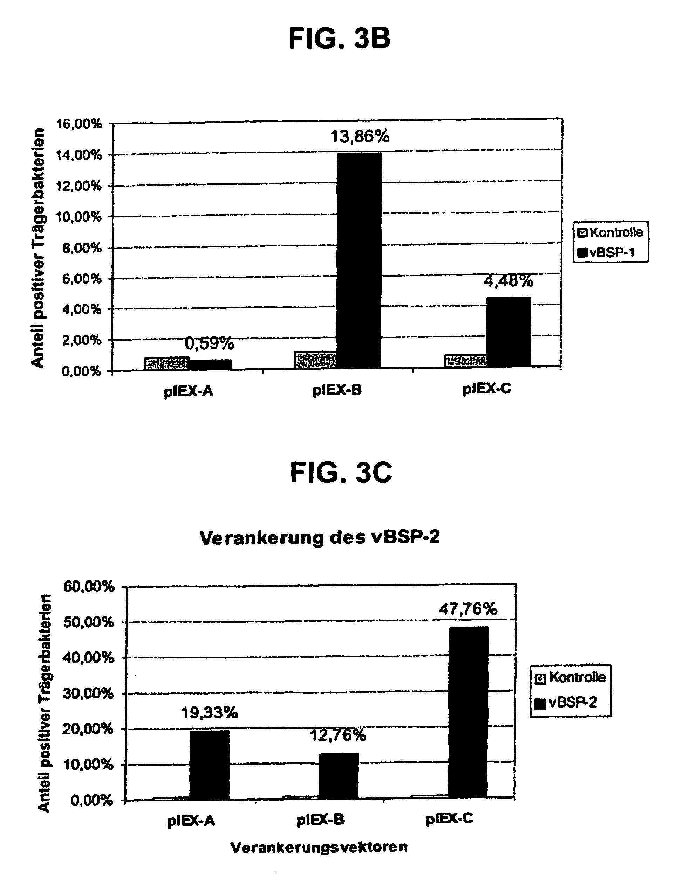

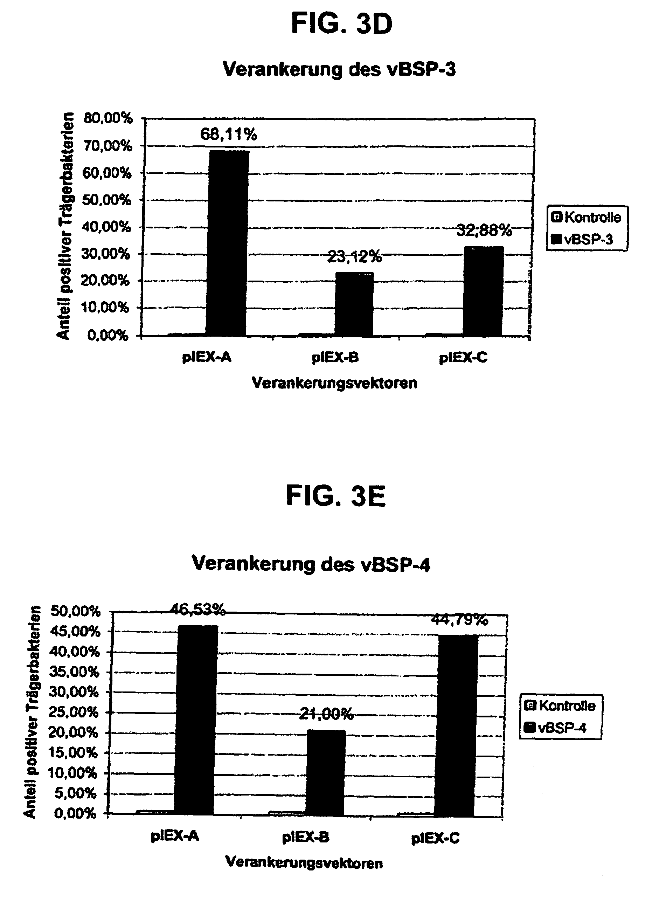

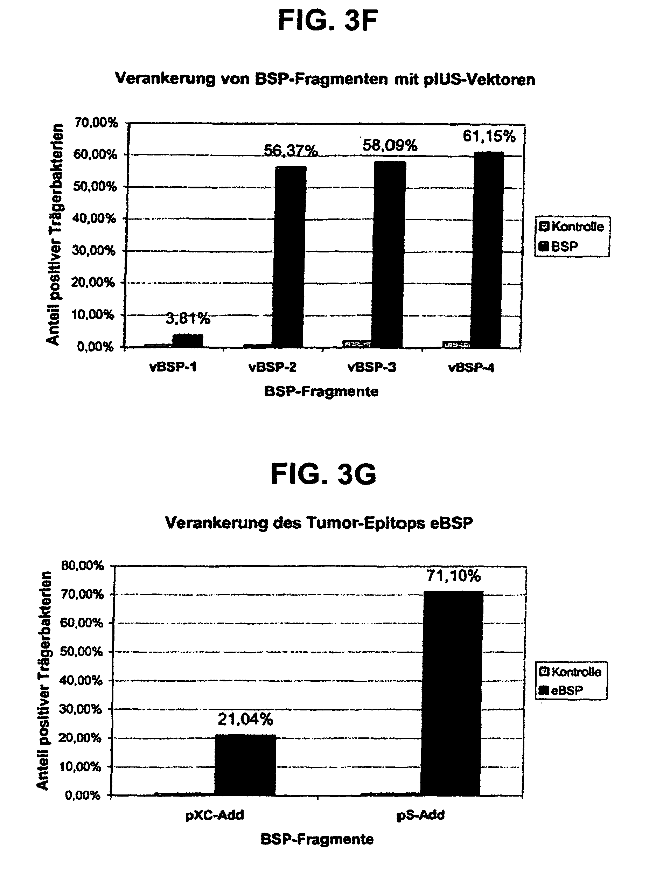

- Fig. 3A: zeichnerische Darstellungen des Anteils positiver Bakterien, die nach FACS-Analyse auf der Oberfläche funktionell verankerte BSP-Fragmente (vBSP-1, -2, -3, -4 bzw. eBSP) tragen, nachgewiesen mit einem für das Tumor-Epitop spezifischen polyklonalen Antiserum (Kaninchen) - als Negativkontrolle wurden Trägerbakterien verwendet, die kein BSP auf der Oberfläche tragen;
- Fig. 3B - 3G: zeichnerische Darstellungen des Anteils positiver Bakterien, die nach FACS-Analyse verankertes vBSP-1, -2, -3, -4 und eBSP tragen für verschiedene Sekretionssignale (plEX-A, -B und -C), Vektoren (plEX, plUS), mit einem Trägerprotein (pXC-Add, PS-Add), nachgewiesen mit monoklonalen Antikörpern gegen den myc-Tag - als Negativkontrolle wurden Trägerbakterien verwendet, die kein rekombinantes Protein auf der Oberfläche trugen;

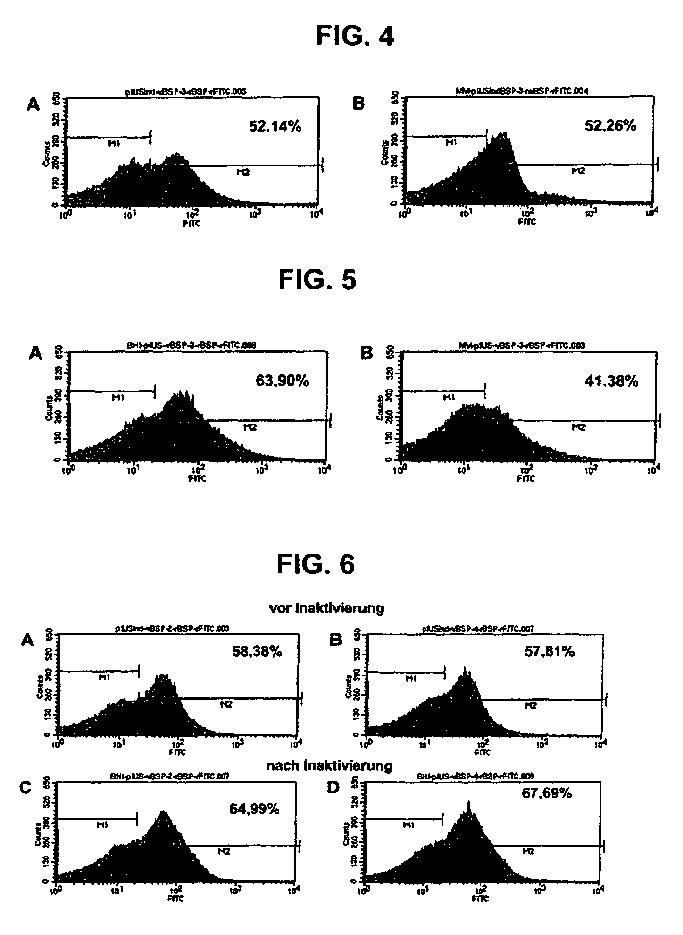

- Fig. 4: Diagramme der Durchflusszytometrie der Trägerbakterien auf funktionell verankertes BSP-Fragment auf der Oberfläche vor ihrer Inaktivierung - A) Verankerung von vBSP-3 bei Anzucht der Trägerbakterien in BHI-Medium; B) Verankerung von vBSP-3 bei Anzucht der Trägerbakterien in Minimalmedium, wobei das Fluoreszenz-aktivierte Zellsorting (FACS) mit polyklonalen Kaninchen-anti-Tumor-eBSP-Antikörpern erfolgte und als Kontrolle Trägerbakterien ohne BSP-Determinanten auf der Oberfläche verwendet wurden;
- Fig. 5: Diagramme der Durchflusszytometrie von Trägerbakterien gemäß Figur 4 nach ihrer Inaktivierung - A) Verankerung von vBSP-3 bei Anzucht in BHI-Medium; B) Verankerung des vBSP3 bei Anzucht in Minimalmedium;
- Fig. 6: Diagramme der Durchflusszytometrie von Trägerbakterien vor und nach ihrer Inaktivierung auf funktionell verankertes BSP-Fragment vBSP2 beziehungsweise vBSP4 - Verankerung von vBSP2 bei Anzucht der Trägerbakterien in BHI-Medium A) vor und C) nach Inaktivierung; Verankerung von vBSP4 bei Anzucht der Trägerbakterien in BHI-Medium B) vor und D) nach Inaktivierung;

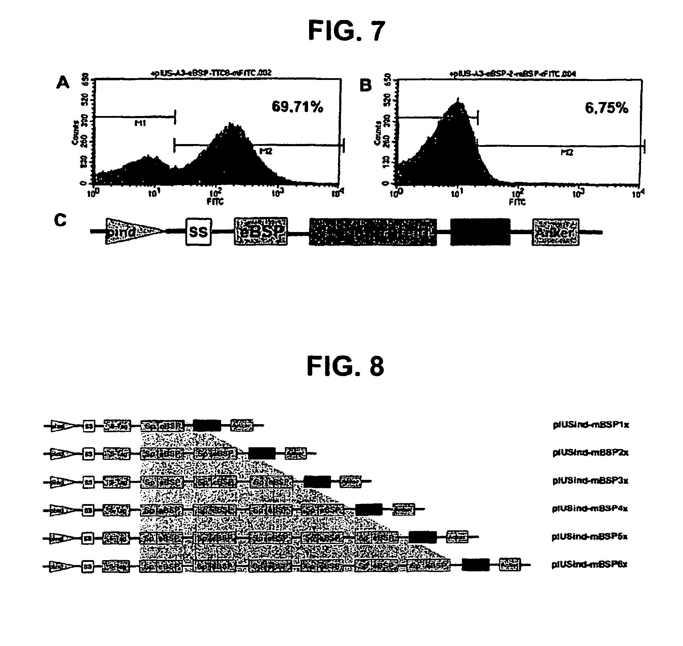

- Fig. 7: eine Durchflusszytometrie von Trägerbakterien auf funktionell verankertes Tumor-eBSP-Epitop, fusioniert mit einem Trägerprotein, nachgewiesen A) mit einem monoklonalen Antikörper gegen das Trägerprotein, B) mit Tumorepitop spezifischen polyklonalen Kaninchen-Antikörpern, und Trägerbakterien ohne rekombinantes Protein auf der Oberfläche als Kontrolle - C) zeichnerische Darstellung der Expressionskassette des eBSP-Konstrukts, SS: Signalsequenz; S-Tag: Immuntag, myc-Tag: Immuntag; Anker: Ankersequenz
- Fig. 8: eine Darstellung der Expressionskassette der Verankerungskonstrukte plUSInd-mBSPIx- 6x: plnd: Promotor; SS: Signalsequenz; S-Tag: Immunologischer Tag; SP: Spacer (SGGGGSA) - SEQ ID NO: 8; myc-Tag: Immuntag; Anker: Ankersequenz

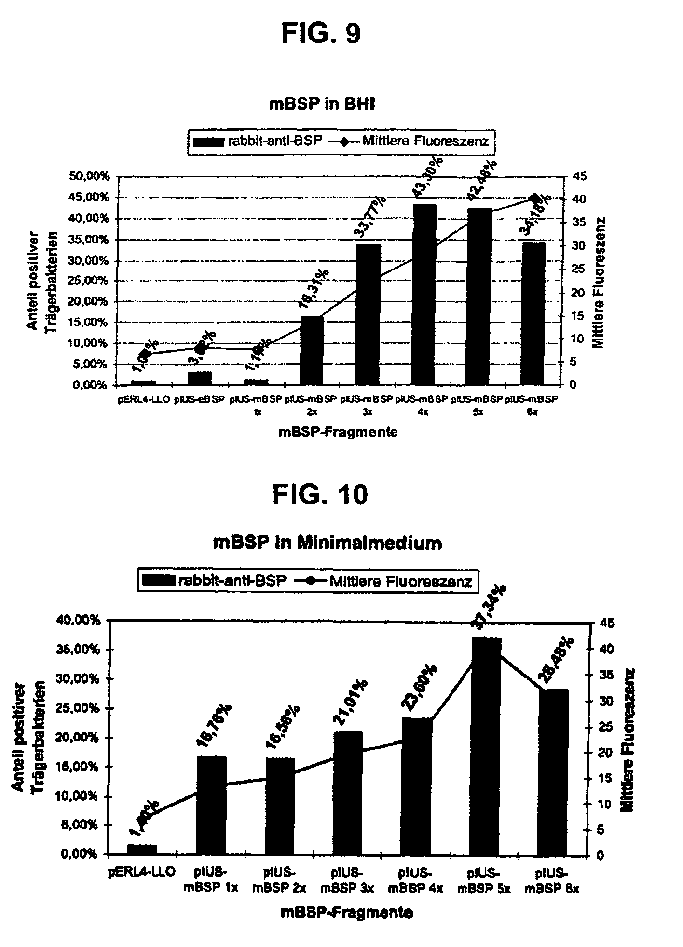

- Fig. 9: eine Durchflusszytometrie von Trägerbakterien mit unterschiedlicher Kopienzahl des BSP-Tumorepitops bei Anzucht in BHI-Medium - funktionell verankertes BSP-Tumorepitop auf der Oberfläche wurde nachgewiesen mit Tumorepitop spezifischen polyklonalen Kaninchen-Antikörpern, und Trägerbakterien ohne BSP-Immuntags auf der Oberfläche (pERL4-LLO) dienten als Kontrolle: plUS-eBSP verankert ein einzelnes und plUS-mBSP-1x- -6x Multimere(1-6) des BSP-Tumorepitops auf der Oberfläche der Trägerbakterien;
- Fig.: 10 Diagramm einer Durchflusszytometrie von in Minimalmedium angezüchteten Trägerbakterien auf funktionell auf der Oberfläche verankerte BSP-Tumorepitope unterschiedlicher Zahl, nachgewiesen mit Kaninchen-Tumor-eBSP-Antiserum, und mit Trägerbakterien ohne BSP-Immuntags auf der Oberfläche (pERL4-LLO) als Kontrolle - pIUS-mBSP-1x- -6x: Verankerung von BSP-Tumorepitop als Multimer (1x - 6x) auf den Trägerbakterien;

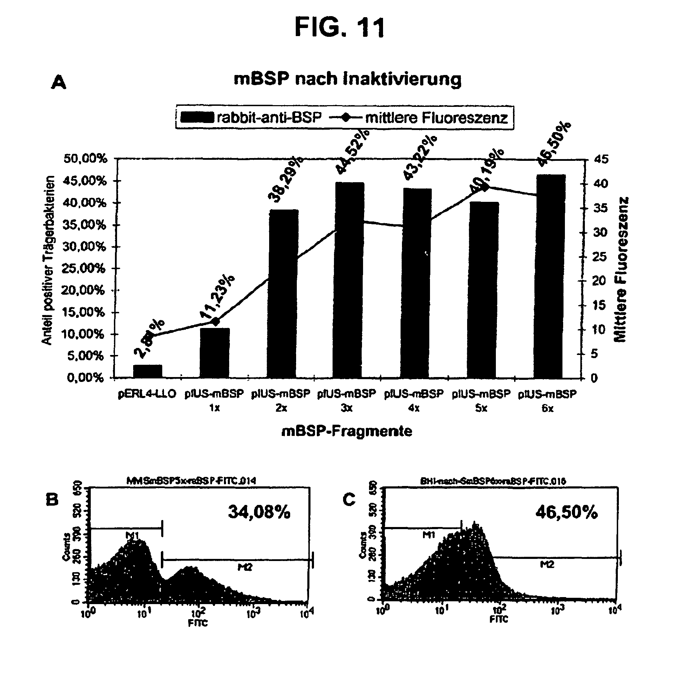

- Fig. 11: Diagramm einer Durchflusszytometrie von Trägerbakterien nach ihrer Inaktivierung auf funktionell auf der Oberfläche verankerte BSP-Tumorepitope unterschiedlicher Zahl, nachgewiesen mit Kaninchen-Tumor-eBSP-Antiserum, und mit Träger-bakterien ohne BSP-Immuntags auf der Oberfläche (pERL4-LLO) - A) pIUS-mBSP-1x - -6x: Verankerungskonstrukte, welche das BSP-Tumorepitop als Multimer (1x - 6x) wie in Figur 8 auf den Trägerbakterien verankern; B) FACS-Diagramm von in Minimalmedium angezüchteten inaktivierten Trägerbakterien, welche das BSP-Tumorepitop als 5er-Multimer auf der Oberfläche verankert tragen; C) FACS-Diagramm von in BHI-Medium angezüchteten, inaktivierten Trägerbakterien, die das BSP-Tumorepitop als 6er-Multimer auf der Oberfläche verankert tragen.

### EINGEHENDE BESCHREIBUNG DER ERFINDUNG

Die osteotrophen Tumore der Prostata, Mamma, Lunge, Niere und Schilddrüse, unterscheiden sich von weniger malignen Tumoren unter anderem dadurch, dass sie BSP exprimierende Zellen enthalten und in das Knochengewebe streuen können. Wenngleich die Metastasierung in Knochen komplex und nicht verstanden ist, so erlaubt das Auftreten von tumorgenen BSP im Serum eine sichere Prognose von Knochenmetastasen. Ferner kann das Vorkommen von tumorgenen BSP in der Knochenmatrix als Maß für den Knochenumbau durch Knochenmetastasen herangezogen werden. BSP aus Tumorzellen besitzt gegenüber endogenem BSP normaler Osteoblasten veränderte posttranslationale Modifikationen. So konnten in Huhn 1gY-Antikörper hergestellt werden, die spezifisch das von Zellen osteotropher Tumore sezernierte BSP erkennen, hier Tumor-eBSP genannt. Diese Antikörper sind im Tiermodell eklatant wirksam gegen induzierte Knochenmetastasen humaner Krebszellen (siehe WO 02/100899). Die erfindungsgemäße therapeutische Zusammensetzung zur Therapie und Prophylaxe von Knochentumoren erzeugt im Patienten eigene Antikörper gegen BSP und insbesondere tumorspezifisches eBSP. Die so induzierten Autoantikörper gegen tumorgenes eBSP interferieren vermutlich mit dem Andockmechanismus der osteotrophen Zellen aus dem Primärtumor. Da aber BSP vom Faktor-H des Komplementsystems gebunden und maskiert wird, entgehen viele eBSP exprimierende Tochterzellen des Primärtumors dem Angriff des Immunsystems und der Nekrose durch das Komplementsystem. Auch die Herbeiführung einer Apoptose wäre von großer Bedeutung. Normalerweise sorgt der Tod von als autoreaktiv erkannten Lymphozyten im Thymus dafür, dass sich die Immunabwehr nicht gegen körpereigene Antigene richtet. Die erfindungsgemäße therapeutische Zusammensetzung umgeht aber diese falschen Selbstschutzmechanismen, da unter anderem spezifisch eine Immunreaktion gegen tumorgenes eBSP aus Tumorzellen erzeugt wird und die Antikörper an ein Epitop des BSP binden, das auch bei seiner Umklammerung durch den Komplementfaktor-H im Serum frei und zugänglich bleibt. Die erzeugte zellvermittelte Zytotoxizität induziert einen nekrotischen oder apoptotischen Tod der Zielzellen. Anders als bei herkömmlichen Therapieansätzen werden also keine Tumor-BSP-spezifischen Antikörper verabreicht, sondern im Patienten durch eine Mischung aus Xeno- und Idio-Immunisierung eine zellvermittelte Immunität erzeugt gegen ein Protein, das kennzeichnend ist für in Knochen metastasierende Tumore und von diesen vermutlich für das Streuen in Knochen benötigt wird.

Die therapeutische Zusammensetzung kann abgetötete oder schwach pathogene Mikroorganismen enthalten, welche eine BSP-kodierende DNA-Sequenz oder ein Fragment hiervon in einem episomalen Vektor integriert enthalten. Die therapeutische Zusammensetzung entspricht somit einem Impfstoff beziehungsweise einem Lebendimpfstoff, in dem BSP-kodierende Fremd-DNA in BSP-Antigene beziehungsweise Tumor-eBSP-Antigene translatiert und exprimiert ist, so dass der Patient Antikörper gegen die präsentierten fremden BSP- und Tumor-eBSP-Antigene entwickelt. Die so erzeugte eigene Immunität gegen Tumor-BSP schützt voraussichtlich gegen ein Andocken von metastasierenden osteotrophen Zellen des Primärtumors im Knochengewebe und verhilft zu einer zellvermittelten Zytotoxizität gegen Tumor-BSP produzierende Tumorzellen. Beide Mechanismen entfalten eine prophylaktische und therapeutische Wirkung gegen Knochenmetastasen.

Weiterhin kann die Wirkung der therapeutische Zusammensetzung verstärkt und moduliert werden durch den Zusatz von Antikörpern, insbesondere Anti-Tumor-BSP-Antikörpern, Liganden, insbesondere RGD-bindende Liganden, Inhibitoren, welche interagieren mit Adhäsionsmolekülen, membran-assoziierten Proteasen oder Rezeptoren, die Chemotaxis vermitteln, beispielsweise Chemokinrezeptoren, sowie Apoptose-induzierende Substanzen wie Antikörpern oder Proteinen und Peptiden, die aus natürlichen oder künstlichen Peptidlibraries gewonnen werden. Besonders erfolgsversprechend erscheinen Peptide aus BSP und eBSP, die durch Kopplung mit beta-Alanin immunogen wurden.

Besonders bevorzugt ist eine therapeutische Zusammensetzung, in der die inaktivierten oder schwach pathogenen Mikroorganismen zu einer Expression von Tumor-eBSP in einer Form führen, dass das Tumor-BSP an der Plasmamembran gebunden ist. Die Plasmamembran kann die Plasmamembran des Mikroorganismus sein oder die Zellmembran von Wirtszellen des Patienten, in denen zielgerichtet die genetische Information des Tumor-BSP eingeführt wurde.

Weiterhin bietet es sich an, die eingeführte BSP-DNA so zu modifizieren, dass nach Expression dem Tumor-BSP ähnliche Moleküle erhalten werden. Die möglichen Stellen für eine Modifikation liegen insbesondere an den Positionen der DNA, die für Aminosäuren kodieren, welche posttranslational N- beziehungsweise O-glykosyliert werden. Durch gerichtete Punktmutationen können so posttranslationale Modifikationen entfernt werden. Tumor-BSP unterscheidet sich nämlich von endogenem BSP vor allem durch ein veränderte oder unvollständige posttranslationale Glykosylierung. Um die Antigenität des klonierten und exprimierten Fragments zu erhöhen, kann es auch mehrfach hintereinander gekoppelt werden.

Die Aminosäuresequenz von humanem BSP enthält vier potentielle N-Glykosylierungsstellen an den Positionen 88 (NTT), 161 (NGT), 166 (NST) und 174 (NGS). Für O-Glykosylierungen ist keine vergleichbare Konsensussequenz bekannt. Alle identifizierten N-Glykan-Strukturen sind sowohl auf dem aus Knochen isolierten BSP als auch auf tumorgenem BSP zu finden. Die Unterschiede liegen jedoch im prozentualen Anteil der jeweiligen Strukturen an den gesamten N-Glykanen. So besteht der Hauptanteil der BSP-N-Glykane im Knochen aus triantennären Strukturen (58%) und z.B. in der "entarteten" EBNA-Zelllinie aus tetraantennären Strukturen (48%). Ferner sind auf dem humanen BSP-Molekül mindestens acht O-Glykosylierungsstellen vorhanden, 5 auf dem Peptid 211-229 (TTTSP ... QVYR) und maximal drei auf dem Peptid zwischen AS 120 und AS 135 mit der Sequenz TGLAA (SEQ ID NO: 3). Hiervon sind im rekombinanten BSP, exprimiert in EBNA-Zellen, die Threonine in der Sequenz DATPGTG (SEQ ID NO: 9) O-glykosyliert. Bei Knochen-BSP erfolgt eine dritte O-Glykosylierung. Bei rekombinantem BSP ist keine dritte Glykosylierungsstelle vorhanden. Vermutlich liegt diese Glykosylierungsstelle auf der TGLAA-BSP-Teilstruktur (SEQ ID NO: 3).

Wegen der vorteilhaften Ergebnisse mit Antikörpern gegen diese Teilstruktur des humanen BSPs wurden diese DNA-Sequenz, gekoppelt mit einer DNA für ein Carrierpeptid und einem Membrananker (poly-His, Internalin-A-Sequenzen), als Fremd-DNA in den Mikroorganismus eingeführt und zur Expression gebracht - im Mikroorganismus selbst oder in somatischen transgenen Wirtszellen des Patienten. Die exprimierten Tumor-BSP-Fragmente wurden im Säugetier als fremd erkannt, da sie auf der Membran eines Mikroorganismus beziehungsweise auf der Zelloberfläche infizierter Wirtszellen lagen. Die so induzierten eigenen Antikörper binden an das BSP der osteotrophen Tumorzellen.

Bevorzugte BSP-Peptidfragment sind wie gesagt
SEQ ID NO: 1
   X-YTGLAAIQLPKKAGD-Z
wobei das markierte Threonin in Tumor-BSP nicht oder unvollständig oder in anderer Form glykosyliert ist. SEQ ID NO: 2 kann wie folgt verändert sein: Position 179 Gly→ Val; Position 252 Val → Ala; Position 254 Glu → Asp; Position 279 Asp → Gly.

In einer Ausführungsform der Erfindung führt die therapeutische Zusammensetzung zur Bildung körpereigener Antikörper gegen ein BSP, dessen posttranslationale Glykosylierung im Bereich der Aminosäuren 120 bis 135 (SWISSPROT: SIAL_HUMAN, Acc.No. P21815) gegenüber normalem BSP aus Knochen verändert oder unvollständig ist. Bevorzugt ist die Induktion körpereigener Antikörper, die ein hBSP-Epitop erkennen, welches die Aminosäuresequenz TGLAA (SEQ ID NO: 3) oder YTGLAA (SEQ ID NO: 4) und optional Zuckergruppen sowie ein Trägermolekül umfasst.

Das erfindungsgemäße Vakzin führt also zu körpereigenen Antikörper gegen tumorgenes BSP. Die so induzierte Immunität schützt somit vor einem Andocken von metastasierenden osteotrophen Tumorzellen im Knochengewebe und verhilft zu einer zellvermittelten Zytotoxizität gegen Tumor-BSP-produzierende Zellen.

Die erfindungsgemäße therapeutische Zusammensetzung ist dem Anwendungsgebiet nach somit besonders geeignet zur Behandlung von Tumoren aus der Gruppe mit Prostata-, Mamma-, Lungen-, Nieren- und Schilddrüsentumoren, Tumorerkrankungen des Blutsystems, des Lymphsystems, des Herz-Kreislauf-Systems, des Nervensystems, des Respirationstraktes, des Verdauungstraktes, des endokrinen Systems, der Haut einschließlich Anhangsgebilde, des Bewegungsapparates und des Urogenitaltraktes.

Die erfindungsgemäßen vakzine können erhalten werden mit einem Verfahren zur Entwicklung von Vakzinen, insbesondere gegen Tumore im Allgemeinen und gegen osteotrophe Tumore, die in Knochen metastasieren. Dieses Verfahren umfasst die Schritte: (i) Identifizieren eines krankheitsrelevanten Proteins wie zum Beispiel BSP; (ii) Erzeugen von Trägerorganismen mit ein oder mehreren ausgewählten Bereichen (Immuntags) des krankheitsrelevanten Proteins, welche die Immuntag auf der Oberfläche exprimiert und verankert tragen; (iii) Testen der vorzugsweise inaktivierten Trägerorganismen, ob sie eine Immunreaktion gegen die Immuntags des krankheitsrelevanten Proteins auslösen; (iv) Untersuchen einer Anzahl Personen, einschließlich erkrankter und gesundeter Patienten, auf Antikörper gegen die Immuntags des krankheitsrelevanten Proteins, beispielsweise durch Reihenuntersuchungen von Blutseren und (v) Wahl der Trägerorganismen mit Immuntags auf der Oberfläche als Impfbakterien unter dem Gesichtspunkt, welche Personen mit welcher Anamnese, einschließlich welche gesundeten Patienten, Antikörper aufweisen, die an ein oder mehreren Immuntags des krankheitsrelevanten Proteins binden. Da die Schritte (i) bis (iii) vergleichsweise rasch und vor allem parallel abgearbeitet werden können und Blutseren für den Schritt (iv) nebst zugehörigen Krankheitsgeschichten (v) millionenfach zur Verfügung stehen, können so gezielt und mit Hilfe statistischer Krankheitsanalyse hilfreiche Impfbakterien mit verankerten Immuntags erzeugt und identifiziert werden. Das Auftreten von natürlichen Antikörpern gegen ein oder mehrere Immuntags des krankheitsrelevanten Proteins im Blutserum ist per se schon ein Indiz, dass das oder die gewählten Immuntags medizinische Relevanz besitzen. Diese Relevanz kann dann durch Vergleich der zugehörigen Anamnesen ermittelt werden. Da Schritt (iv) im Analyseautomaten an massenhaft vorhandenen Blutseren erfolgen kann, können bereits durch statische Analyse relevante von nicht-relevanten Antigenabschnitten, ohne jeweilige medizinische Anamnese, geschieden werden. Bei der Suche nach tumorrelevanten Immuntags werden besonders die Seren von Patienten mit Spontanheilungen oder mildem Krankheitsverlauf zu prüfen sein.

### BEISPIELE

### Beispiel 1 Expressionsvektors und Mikroorganismen für die Immunisierung

Die cDNA für humanes BSP (SEQ ID NO 2) bzw. die nachgenannten BSP-Fragmente wurde in die Polyklonierungstelle eines episomalen pSOG-Shuttlevektors kloniert (Machner et al., J. Biol. Chem., 2001, 276(43), 40096; (Fisher L.W. et al., J. Biol. Chem., 1990, 265(4), 2347-51). Um eine Verankerung der klonierten heterologen BSP-Antigene auf der Oberfläche von Listerien zu erreichen, wurde in den E. coli/Listerien-Shuttlevektor (Erythromycin-Resistenz, ColE1 origin of replication, Gram-positives Minimalreplikon für Gram-positive Bakterien) eine Expressions- und Verankerungskassette integriert. Alle Komponenten der Expressions- und Verankerungskassette wurden mittels PCR aus den jeweiligen Organismen amplifiziert und in den Shuttlevektor kloniert. Die relative Anordnung der einzelnen Komponenten der Expressions- und Verankerungskassette sind in Figur 8 gezeigt.

Für die Expression der rekombinanten Peptide wurde der Promotor (pind) des *Listeriolysin*-Gens aus *Listeria monocytogenes* verwendet (Mengaud et al., Infect. Immun. 1989, 57:3695). Der Promotor wurde zusammen mit dem 5'-untranslatierten Bereich des Listeriolysin-Gens (Accession No. NC003210) upstream zu einer Nukleotidsequenz kloniert, die für das Signalpeptid (SS) des Listeriolysins (LLO - Proteinsequenz in Swiss-Prot: Q724L1; Nukleotidsequenz, Genbank Accession Nos. P13128, X15127) kodiert. Die Identifizierung der Signalsequenzen erfolgte nach Literaturangaben und mit Hilfe des Programms SignaIP vs2.0 (Nielsen et al., Protein Engineering, 1999, 12:3). Das in der Regel ca. 25 Aminosäuren lange Signalpeptid transportiert die rekombinanten Proteine über die Zellmembran des Trägerbakteriums und wird dann abgespalten. Upstream und downstream der BSP-Epitope kodierenden Gene wurden in einer Ausführungsform zudem Nukleotidsequenzen eingefügt, welche zwei immunologische Tags (S-Tag von Novagen, Darmstadt; upstream, myc-Tag von NatuTec GmbH, Frankfurt; downstream) kodieren. Das S- und das myc-Tag erleichterten nach Expression die immunologische Charakterisierung der Verankerung der BSP-Epitope auf der Oberfläche der Listerien. Um eine sichere Verankerung der rekombinanten heterologen Antigendeterminaten auf den Listerien zu erreichen, wurde downstream des myc-Tags eine Sequenz aus dem Internalin A (InlA) von L. *monocytogenes* integriert. Der eingefügte Anker umfasste die Positionen 677-800 des InlA (Swiss-Prot: Q723K6).

Zwischen den beiden immunologischen Tags wurden dann Nukleotidsequenzen kloniert, welche für die BSP-Tumorepitope als Monomer oder als Polymer mit 2 bis 6 Kopien des BSP-Tumorepitops kodieren. Das BSP-Tumorepitop hat die Aminosäuresequenz -EDATPGTGYTGLAAIQLPKKAG- (eBSP) (SEQ ID NO: 10). Die einzelnen Tumorepitope sind durch einen kurzen Spacer der Aminosäuresequenz -SGGGGSA- (Sp) (SEQ ID NO 8) miteinander verbunden. Tumorepitop und Spacer wurden mit PCR amplifiziert und mit Standardverfahren in das Verankerungsplasmid pIUSind kloniert.

Die Konstrukte führten am Beispiel pIUSind-mBSP1x - 6x in Listerien somit zur Expression von Fusionspeptiden, die aus einem N-terminalen Signalpeptid, einem S-Tag, einem (pIUSind-mBSP1x) oder mehreren BSP-Epitopen (pIUSind-mBSP2x - 6x), einem myc-Tag und einer Ankerstruktur bestanden. Das Signalpeptid bewirkte, dass das Fusionspeptid aus dem Trägerbakterium geschleust wird, während die Ankerstruktur des Internalin-As für eine "kovalente" Verankerung des BSP-Epitops in der bakteriellen Zellwand sorgte. Der korrekte Leserahmen wurde durch DNA-Sequenzierung und Expression überprüft; das Fusionspeptid wurde von Anti-hBSP-Antikörpern erkannt und auch vom therapeutischen IgY-Antikörper. Bei der DSMZ (German National Resource Centre for Biological Material, Braunschweig) wurden für die vorliegende Patentanmeldung hinterlegt die beiden Listeria pIUSind-mBSP5x (**DSM 18306**) und Listeria pIEx-A-vBSP3 (**DSM 18305**), welche die Vektoren enthalten.

### Beispiel 2 Therapeutische wirksame BSP-Antigenstrukturen

Es wurden die Epitope von therapeutisch aktiven anti-BSP-1gG und -1gY kartiert, um die von ihnen gebundenen Antigene beziehungsweise Haptene für die Immunisierungsversuche gegen tumorgenes BSP näher zu charakterisieren.

**TABELLE 1**

| *BSP-Epitope therapeutisch aktiver anti-BSP-1gG und -1gY-Globuline* | | | |
|---|---|---|---|
| Lage der Peptidteilstruktur im BSP | Aminosäuresequenz | Reaktionsstärke Huhn - Kaninchen | |
| (Position, einschließlich Leader) | | IgY | IgG |
| 112-123 - SEQ ID NO:11 | LeuGlyTyrGlyGluAspAlaThrProGlyThrGly | - | ? |
| 216-227 - SEO ID NO:12 | GluThrGlyGlyGinGlyLysGlyThrSerLysThr | - | ? |
| 300-311 - SEQ ID NO:13 | PheLysGlyGlnGlyTyrAspGlyTyrAspGlyGln | - | ? |
| 130-144 - SEQ ID NO:14 | IleGlnLeuProLysLysAlaGlyAspIleThrAsnLysAlaThr | +/- | + |
| 124-138 - SEQ ID NO:01 | TyrThrGlyLeuAlaAlalleGlnLeuProLysLysAlaGlyAsp | - | ++ |
| 137-151 - SEQ ID NO:15 | GlyAspIleThrAsnLysAlaThrLysGluLysGluLysGlu-SerAspGlu | - | + |
| 280-317 - SEQ ID NO:16 | SerGluAsnGlyGluProArgGlyAspAsnTyrArgAlaTyr-GluAspGluTyrSerTyrPheLysGlyGlnGlyTyrAspGly-TyrAspGlyGlnAsnTyrTyrHisHisGln | ++ | + |
| Humanes Knochen-BSP | | +++ | +++ |

Die Ergebnisse zeigen, dass die bekannten Hühnerantikörper bevorzugt an die C-terminale Sequenz des BSP binden, während die Kaninchenantikörper über einen größeren Bereich binden. Therapeutisch relevante Bereiche des humanen Bonesialoproteins sind danach:

| | |
|---|---|
| SEQ ID NO: 1 | |
| TyrThrGlyLeuAlaAlalleGlnLeuProLysLysAlaGlyAsp | (Position 124-138) |
| SEQ ID NO: 3 | |
| ThrGlyLeuAlaAla | (Position 125-130) |
| SEQ ID NO: 4 | |
| TyrThrGlyLeuAlaAla | (Position 124-130) |
| SEQ ID NO: 6 | |
| TyrGluSerGluAsnGlyGluPro**ArgGlyAsp**AsnTyrArgAlaTyrGluAsp | (Position 278-295) |
| SEQ ID NO: 7 | |
| LeuLysArgPheProValGlnGlyGly | (N-Terminus) |

Für eine sekundäre Eingrenzung der tumorrelevanten BSP-Strukturen wurden größere Fragmente aus humanem BSP kloniert und in Bakterien exprimiert, nämlich
- **vBSP-1:**: 301 Aminosäuren der hBSP-Sequenz von Position 17 bis 318, entsprechend der vollständigen Sequenz von humanem BSP (ohne Signalsequenz).
- **vBSP-2:**: 200 Aminosäuren von Position 57 bis 257 - vBSP-2 enthält keine Tyrosinreichen Regionen und enthält auch keine RGD-Sequenz. vBSP-2 beginnt direkt nach der ersten Tyrosin-reichen Region und endet unmittelbar vor der zweiten Tyrosin-reichen Region.
- **vBSP-3:**: 234 Aminosäuren von Position 84 bis 318. vBSP-3 enthält nicht die erste Glutamat-reiche Region und erstreckt sich bis zum C-Terminus des BSP.
- **vBSP-4:**: 174 Aminosäuren von Position 84 bis 257. vBSP-4 ist verkürzt um die erste Glutamat-reiche Region und alle Tyrosin-reichen Regionen.
- **eBSP:**: 22 Aminosäuren (-EDATPGTGYTGLAAIQLPKKAG- (eBSP) - SEQ ID NO: 10) von Position 115 bis 137, umfassend ein als Tumor-Epitop erkannte antigene Determinante des BSP.

Figur 2 zeigt die Lage der genannten Fragment im BSP-Protein zu den anderen als relevant beschriebenen Strukturen

### Beispiel 3 Generierung der für die Verankerung nötigen BSP-Konstrukte

Die DNA-Sequenzen der BSP-Fragmente vBSP-1, -2, -3, -4 und eBSP (Beispiel 2, Figur 2) wurden mittels PCR amplifiziert und in die beschriebenen Shuttlevektoren kloniert. Die klonierten Gene wurden durch Sequenzierung verifiziert und in verschiedene Sekretions- und Verankerungsvektoren kloniert. Die BSP-Fragmente vBSP-1, -2, -3 und -4 wurden am C-Terminus mit einem immunologischen Tag (myc-Tag) fusioniert, um die Detektion der verankerten Fragmente zu erleichtern. Das nur 22 Aminosäuren umfassende Fragment mit eBSP wurde an den N-Terminus eines bakteriellen Trägerproteins fusioniert, um zu verhindern, dass das eBSP wegen seiner geringen Größe nicht aus der bakteriellen Zellwand herausragt oder von den Zellwandmolekülen maskiert wird. Im Einzelnen wurden die in Tabelle 2 aufgeführten Klone generiert:.

**TABELLE 2**

| **Verankerungskonstrukt** | **Verankerungsvektor** | **BSP-Fragment** | **Trägerprotein** |
|---|---|---|---|
| pIEX-A-vBSP1 | pIEX-A | vBSP1 | nein |
| pIEX-A-vBSP2 | pIEX-A | vBSP2 | nein |
| pIEX-A-vBSP3 | pIEX-A | VBSP3 | nein |
| pIEX-A-vBSP4 | pIEX-A | vBSP4 | nein |
| pIEX-B-vBSP1 | pIEX-B | vBSP1 | nein |
| pIEX-B-vBSP2 | pIEX-B | vBSP2 | nein |
| pIEX-B-vBSP3 | pIEX-B | VBSP3 | nein |
| pIEX-C-vBSP1 | pIEX-C | vBSP1 | nein |
| pIEX-C-vBSP2 | pIEX-C | VBSP2 | nein |
| pIEX-C-vBSP3 | pIEX-C | VBSP3 | nein |
| pIUS-vBSP1 | pIUS | vBSP1 | nein |
| pIUS-vBSP2 | pIUS | VBSP2 | nein |
| pIUS-vBSP3 | pIUS | vBSP3 | nein |
| PXC-Add-eBSP | pXC-Add | eBSP | ja |
| pS-Add-eBSP | pS-Add | eBSP | ia |

Alle Klone wurden in *E*. coli generiert und anschließend in Trägerbakterien transformiert.

Es wurde dann untersucht, ob sich die einzelnen BSP-Fragmente in den Trägerbakterien exprimieren, sekretieren und funktionell verankern lassen. Unter einer "funktionellen Verankerung" der BSP-Fragmente war hinsichtlich der geplanten Anwendung der Impfbakterien verlangt, dass die BSP-Fragmente in den Trägerbakterien exprimiert, ausgeschleust und schließlich auf der Bakterienoberfläche kovalent verankert wurden. Die verankerten BSP-Fragmente enthielten unter anderem das BSP-Tumor-Epitop, welches vom Tumor-Epitop-spezifischen Antiserum erkannt wurde. Durch diesen Schritt wurde sichergestellt, dass das BSP-Tumor-Epitop tatsächlich auf den Oberflächen der Trägerbakterien präsentiert war und bei einem Einsatz der Impfbakterien zur aktiven Immunisierung zu einer Tumor-Epitop-spezifischen Immunantwort führen konnte.

Um eine funktionelle Verankerung der BSP-Fragmente zu erreichen, wurden die generierten BSP-Verankerungskonstrukte in die Trägerbakterien transformiert und die jeweiligen BSP-Fragmente auf den Bakterien verankert. Der Nachweis der funktionellen Verankerung erfolgte im Durchflusszytometer mit dem polyklonalen Antiserum "Anti-Human Bone Sialoprotein (aa 108-122) Antibody" der Firma Immundiagnostik (Cat.-No. A4219.2, Lot H3150503). Dieses polyklonale Antiserum detektierte das Tumor-Epitop des BSP. Ein positiver Nachweis mit diesem Antiserum bewies damit die "funktionelle Verankerung" der einzelnen BSP-Fragmente als heterologe Listerien-Oberflächenantigene zur aktiven Immunisierung.

Die Vorarbeiten ergaben, dass die nachstehenden bezeichneten BSP-Fragmente in Bakterien und auch in *Listerien* funktionell *exprimiert* wurden. Zudem wurden sie aus den Trägerbakterien *sekretiert* und auf ihnen funktionell *verankert*. Es wurden also ausgewählte Fragmente von hBSP auf *L.monocytogenes* gekoppelt und die Bakterien darauf getestet, ob sie eine Immunreaktion gegen das klonierten BSP-Fragment, das tumorgene BSP-Epitop und schließlich gegen die Tumorzellen hervorrufen. Hierzu wurden zunächst Signalsequenzen identifiziert, welche die identifizierten BSP-Fragmente effizient aus den Bakterien sekretieren, aber nur so, dass sie die BSP-Fragmente auf den Listerien verankert blieben. Die Verankerungseffizienz für die gewählten BSP-Fragmente sollten bei mehr als 40% liegen - wenn der Nachweis mit Antikörpern gegen die immunologischen Tags erfolgte. Ferner wurden Bedingungen identifiziert, bei denen die gewählten BSP-Fragmente stabil und reproduzierbar auf den Bakterien verankert bleiben. Schließlich wurden die Bedingungen bestimmt für die Propagierung und Inaktivierung der BSP-Bakterien für die Immunisierungen. Insgesamt lag die Verankerungseffizienz bei ≥ 40 %. Der Fachmann wird aber die optimalen Anzuchtbedingungen und die am besten geeigneten Verankerungssequenzen für jeden Bakterienstamm in üblicher Weise zu bestimmen haben. Im Einzelnen wurden folgende Fragmente aus humanem BSP auf den Trägerbakterien verankert. Die Verankerungseffizienzen sind Tabelle 3 zu entnehmen.

**TABELLE 3**

| *Verankerungseffizienzen* | | |
|---|---|---|
| **BSP-Fragment** | **Vektor** | **Verankerungseffizienz** |
| vBSP-1 | PIEX-B | >13% |
| vBSP-2 | PIUS | >56% |
| vBSP-2 | PIEX-C | >47% |
| vBSP-3 | PIUS | >58% |
| vBSP-3 | PIEX-A | >68% |
| vBSP-4 | PIUS | >61% |
| vBSP-4 | PIEX-A | >46% |
| eBSP | PS-Add | >71% |

Die Ergebnisse dieser Versuche sind im direkten Vergleich bezüglich Fragment, Verankerungsvektor, Anzuchtbedingungen in den Figuren 3A-G graphisch zusammengefasst. Um BSP-Fragmente auf Trägerbakterien zu verankern, kamen zwei verschiedene Vektorsysteme zum Einsatz. Während die *pIEX-Vektorsysteme* für die Verankerung von heterologen Proteinen auf Trägerbakterien bei Anzucht in einem *vollsynthetischen Kulturmedium* konzipiert waren, eigneten sich die *pIUS-Vektoren* für die Verankerung von Proteinen bei Anzucht der Trägerbakterien in *Komplexmedien*.

Das BSP-Fragment vBSP-1 wurde am effizientesten mit dem Sekretionssignal des Vektorsystems pIEX-B auf Trägerbakterien verankert. Die Verankerungseffizienz des vBSP-1 war mit 13,86% (siehe Figur 3B) vergleichsweise gering. Dies erklärt sich am wahrscheinlich dadurch, dass vBSP-1 in den Trägerbakterien nur mit verhältnismäßig niedrigen Expressionsraten produziert wird. Studien anderer Labors haben gezeigt, dass in prokaryonten Expressionssystemen Voll-Längen-BSP (∼ vBSP-1) meist überhaupt nicht produziert wird. Da im verwendeten System Voll-Längen-BSP (vBSP-1) auf den Trägerbakterien verankert wurde, kann man davon ausgehen, dass die Verankerung auf Bakterien eine stabilisierende Wirkung auf das Voll-Längen-BSP hat. Danach wurden die BSP-Fragmente vBSP-1, -2, -3 und -4 in den Trägerbakterien funktionell exprimiert, aus den Trägerbakterien sekretiert und auch auf den Trägerbakterien funktionell verankert. Schließlich konnte auch das Tumor-Epitop eBSP als Fusionsprotein in den Trägerbakterien exprimiert, sekretiert und funktionell verankert werden. Der Nachweis der funktionellen Verankerung erfolgte unter anderem auch mittels FACS und monoklonalen Antikörpern, die den an den C-Terminus der BSP-Fragmente fusionierten immunologischen Tag (myc-Tag) erkennen.

Für eine optimierte Sekretion und Verankerung heterologer Proteine aus bzw. auf den Trägerbakterien wurden abhängig von den spezifischen biochemischen Eigenschaften der zu verankernden Proteine unterschiedliche Signalsequenzen benötigt. In einer Versuchsserie wurden daher verschiedene Signalsequenzen auf ihre Eignung zur Sekretion und Verankerung der BSP-Fragmente untersucht.

Für die weiteren Versuche wurden vor allem die hBSP-Fragmente vBSP-3 und eBSP untersucht. Die Struktur und Lage dieser Fragmente im humanem BSP ist Figur 2 zu entnehmen. Diese beiden BSP-Determinanten sind besonders vorteilhaft, da vBSP-3 neben dem Tumorepitop und zwei C-terminalen Tyrosin-reichen Regionen auch das RGD-Motiv enthält. Dem RGD-Motiv beziehungsweise den immunologischen Reaktivitäten gegen das RGD-Motiv werden eine Funktion bei der Induktion der Apoptose zugeschrieben. Bei Verwendung des eBSP beziehungsweise von Multimeren des eBSP wurden neben dem eigentlichen Tumorepitop keine weiteren BSP-Strukturen als Antigen verankert. Dies reduziert das Risiko, dass die Immunisierung zu Reaktivitäten gegen gesunde Zellen führt, die natives BSP exprimieren.

### Beispiel 4 Verankerung von hBSP-Fragmenten

### Verankerung von vBSP-3

Es wurden die Bedingungen für eine stabile und reproduzierbare Verankerung des vBSP3-Fragments bestimmt. Hierzu wurden folgende Konstrukte auf ihre Eignung für eine funktionelle Verankerung des vBSP3-Fragmentes auf den Trägerbakterien getestet. Der Nachweis der funktionellen Verankerung erfolgte im Durchflusszytometer mit polyklonalen Antikörpern "Anti-Human-Bonesialoprotein aa108-122 (Cat.-No. A4219.2, Lot H3150503) der Firma Immundiagnostik AG, Bensheim, DE. Diese polyklonalen Antikörper erkennen ein Epitop auf BSP (fortan Tumorepitop genannt), welches nur auf Bonesialoprotein aus Tumorzellen vorhanden ist. Ein positiver Nachweis mit diesen Antikörpern steht für funktionelles BSP-Fragment als Antigen für eine aktive Immunisierung gegen tumorgenes BSP.

**TABELLE 4**

| *Verankerungskonstrukte* | | |
|---|---|---|
| **Verankerungskonstrukt** | **Verankerungsvektor** | **BSP-Fragment** |
| pIEX-A-vBSP3 | pIEX-A | VBSP-3 |
| pIEX-B-vBSP3 | pIEX-B | vBSP-3 |
| pIEX-C-vBSP3 | pIEX-C | VBSP-3 |
| PIUS-VBSP3 | PIUS | VBSP3 |

Die besten Ergebnisse wurden mit dem Verankerungskonstrukt pIEX-A-vBSP-3 und pIUS-vBSP-3 erreicht (siehe Figuren 3D und 3F). Durch Variation der Anzucht- und Verankerungsbedingungen konnte eine Optimierung der funktionellen Verankerung des vBSP3 sowohl bei Anzucht in BHI-Medium (Hirn-Herz-Medium) als auch bei Anzucht in einem synthetischen Minimalmedium erreicht werden; siehe Figuren 4 und 5). Die Verankerungseffizienz von über 52% bei Anzucht in BHI- als auch in Minimalmedium waren ausgezeichnet (siehe Figur 4A und B). vBSP3-Bakterien konnten damit sowohl in BHI-Medium als auch in Minimalmedium angezüchtet werden. Das BHI-Medium bot die Vorteile eines schnellen Wachstums und einer hohen Bakteriendichte im Kulturmedium, so dass darin in kurzer Zelt eine hohe Anzahl an Impfdosen hergestellt werden können. Im Gegensatz dazu wuchsen vBSP3-Bakterien in Minimalmedium langsamer und weniger dicht. Dafür besteht bei Anzucht im synthetischen Minimalmedium keine Gefahr der Kontamination der Impfdosen mit BSE beziehungsweise TSE. Dies kann ein entscheidender Vorteil bei der zukünftigen Verwendung von vBSP3-Bakterien im Menschen sein.

### Beispiel 5 Inaktivierung der rekombinaten Listerien bei Erhalt der Verankerung von hBSP-Fragmenten

Bakterien, die rekombinante Proteine auf ihren Oberflächen verankert tragen sind gentechnisch veränderte Organismen (GVOs). Die Immunisierung mit GVOs ist selbst im Tiermodell nur unter sehr aufwendigen, sicherheitstechnischen Vorkehrungen möglich. Inaktivierte Impfbakterien hingegen sind keine GVOs mehr und können daher wie eine konventionelle Vakzine behandelt werden.

Es wurden daher Bedingungen identifiziert, mit denen die Trägerbakterien sicher inaktiviert werden können, gleichzeitig aber das vBSP3 funktionell auf den Bakterienoberflächen verankert bleibt. Figur 5 zeigt die Effizienz der Verankerung von vBSP3 auf inaktiierten Impfbakterien bei der Anzucht in BHI (Fig. 5A) und Minimalmedium (Fig. 5B). Wie aus Figur 5 hervorgeht, ließen sich die Trägerbakterien durch Zusatz von Formalin sicher inaktiieren, wobei das vBSP3 funktionell verankert auf den Oberflächen der abgetöteten Bakterien bestehen blieb und von Tumorepitop-spezifischen Kaninchen-Antikörpern erkannt wurde. Die Verankerungseffizienz des vBSP3 auf den inaktivierten Trägerbakterien betrug ca. 64% bei Anzucht in BHI-Medium und ca. 41% bei Anzucht in Minimalmedium (siehe Figur 5), was für derartige Epitope hervorragende Werte darstellte.

### Beispiel 6 Verankerung der BSP-Fragmente vBSP2 und vBSP4

Das vBSP3-Fragment enthält neben einer Glutamat-reichen Region die zwei C-terminalen Tyrosin-reichen Regionen und das RGD-Motiv des BSP. Im Gegensatz dazu fehlen den BSP-Fragmenten vBSP2 und vBSP4 Tyrosin-reiche Regionen und das RGD-Motiv (siehe Figur 2). Um die Option die Bedeutung einzelner funktioneller Regionen des BSP auf die Immunantwort des geimpften Individuums charakterisieren zu können, wurden vBSP2- und vBSP4-Impfbakterien etabliert.

Figur 6 zeigt die Effizienz der Verankerung der BSP-Fragmente vBSP2 (A und C) und vBSP4 (Figur 6, B und D) auf Trägerbakterien vor und nach der Inaktivierung, wie sie unter optimierten Versuchsbedingungen erreicht werden. Wie Figur 6 zeigt, konnten inaktivierte Impfbakterien hergestellt werden, die Verankerungseffizienzen von fast 65% (vBSP2-Impfbakterien) beziehungsweise fast 68% (vBSP4-Impfbakterien) besaßen. Somit eigneten sich die produzierten vBSP2- und vBSP4-Impfbakterien gleichfalls hervorragend für Immunisierungsversuche.

### Beispiel 7 Verankerung des BSP-Tumorepitops auf Listerien

Die anfänglich generierten Verankerungskonstrukte für das eBSP-Tumorepitop gaben eine Verankerungseffizienz von lediglich etwa 6 bis 7%, gemessen mit Antikörpern gegen eBSP (siehe Figur 7B). Die geringe Verankerungseffizienz war aber nicht das Resultat einer niedrigen Verankerungseffizienz von eBSP als Fusionsprotein auf den Bakterien. Dies zeigte die gefundene Verankerungseffizienz von nahezu 70%, wenn diese mit einem Antikörper gegen für das Trägerprotein bestimmt wurde. Das BSP-Tumorepitop wurde aber als Fusionsprotein mit dem Trägerprotein auf den Bakterien verankert. Folglich muss das BSP-Tumorepitop im Verhältnis gleichermaßen wie das Trägerprotein auf den Bakterien vorliegen und die Verankerungseffizienzen von Trägerprotein und BSP-Tumorepitop gleich sein. Trotzdem wurde eine Verankerungseffizienz von fast 70% für das Trägerprotein gemessen, während die Verankerung des BSP-Tumorepitops nur knapp 7% betrug (siehe Figur 7A). Wahrscheinlich war die Affinität des Tumor-BSP-spezifischen Antikörpers deutlich geringer als die Affinität des monoklonalen Antikörpers gegen das Trägermolekül oder das Tumorepitop wurde wegen seiner geringen Größe von der bakteriellen Zellwand maskiert und nicht vom Antikörper erkannt. Es konnte auch sein, dass das Tumorepitop vom Trägerprotein maskiert wurde. Bei einem singulären kleinen Epitop wie dem BSP-Tumorepitop besteht nämlich stets die Gefahr, dass dieses nicht vollständig zugänglich ist. Die Immunogenität derartiger Impfbakterien bezüglich des Tumorepitops wäre dann gering. Um die Wahrscheinlichkeit eines Impferfolges mit eBSP-Impfbakterien zu erhöhen, wurde daher das Tumorepitop als Multimer auf Trägerbakterien verankert

### Beispiel 8 Generierung von Konstrukten zur Verankerung multimerer BSP Tumorepitope

Generiert wurden Verankerungskonstrukte, die eBSP-Tumorepitop eins- bis sechsfach auf den Oberflächen von Trägerbakterien verankern können (Figur 8). Um die freie Beweglichkeit der einzelnen BSP-Tumorepitope zu gewährleisten, wurde zwischen die einzelnen Tumorepitope ein Spacer aus 7 Aminosäuren (-SGGGGSA-) (SEQ ID NO: 8) kloniert. Die vergleichende Analyse der Verankerung multimerer BSP-Epitope bei Anzuchten in BHI-Medium (Figur 9) und Minimalmedium (Figur 10) zeigte, dass die messbare Verankerungseffizienz multimerer BSP-Epitope von 1-fach bis 5-fach von unter 10% auf über 40% ansteigt, bei der Verankerung eines 6-fach eBSP-Multimers aber wieder leicht abfällt. Im Gegensatz dazu stieg, zumindest bei Anzuchten in BHI-Medium, die mittlere Fluoreszenz der Impfbakterien auch bei der Verankerung eines 6x-eBSP-Tumorepitops weiter an (Figur 9). Dies legt nahe, dass bei der Verankerung eines 6x-eBSP-Tumorepitops zwar weniger Bakterien in der Kultur Tumorepitope auf ihrer Oberfläche verankert trugen, diese aber offensichtlich eine höhere Anzahl an Tumorepitopen pro Bakterienzelle verankern als Impfbakterien mit 4x- oder 5x-BSP-Tumorepitopen. Welche der Impfbakterien 4x-eBSP, 5x-eBSP oder 6x-eBSP die höchste Immunogenität besitzt, werden die Tierversuche zeigen.

Repetitive DNA-Bereiche, die eine 100%ige Sequenzhomologie aufweisen, sind oft instabil. Obwohl die generierten Konstrukte pIUS-mBSP1x - 6x repetitive DNA-Abschnitte besaßen, wurden in den Versuchen keine Instabilitäten der Verankerungskonstrukte beobachtet. Somit konnten Impfbakterien hergestellt werden, die stabil waren und deren messbare Verankerungseffizienz auf über 40% gesteigert werden konnte (siehe Figur 9). Impfbakterien, die das BSP-Tumorepitop als 3er-, 4er-, 5er und 6er-Multimer auf ihren Oberflächen verankert trugen, zeigten auch nach Inaktivierung eine hohe messbare Verankerungseffizienz von über 40% (siehe Figur 11). Die Verankerungsraten lagen bei Anzuchten in BHI-Medium tendenziell stets etwas höher als bei Anzuchten in Minimalmedium. Trotzdem konnten Impfbakterien mit BSP-Tumorepitop-Multimeren sowohl in BHI-Medium (Figur 11, C) als auch in Minimalmedium (Figur 11, B) gezüchtet werden. Damit wurden Impfbakterien generiert, die auf ihren Oberflächen BSP-Fragmente oder Multimere des Tumorepitops verankert tragen. Wie aus der nachstehenden Tabelle ersichtlich ist, konnten für alle Impfbakterien Verankerungsraten von zum Teil deutlich über 40% erreicht werden.

**TABELLE 5**

| *Verenkerungsfragmente und -effizienzen* | | |
|---|---|---|
| | VERANKERUNGSEFFIZIENZ | |
| BSP-FRAGMENT | VOR INAKTIVIERUNG | NACH INAKTIVIERUNG |
| vBSP-2 | 58.4% | 65,0% |
| vBSP-3 | 52.3% | 63,9% |
| vBSP-4 | 57.8% | 67,8% |
| vBSP-5x | 42.5% | 40,1% |
| vBSP-6x | 34.2% | 46,5% |

Damit standen Impfbakterien als Vakzin zur Verfügung, die BSP-Teilfragmente (vBSP2, vBSP3 oder vBSP4) oder das BSP-Tumorepitop als Multimer in hoher Menge als Antigen auf der bakteriellen Oberfläche verankert trugen, somit eine Immunreaktion gegen BSP hervorrufen.

### Beispiel 9 - Therapeutische wirksamkeit

Um eine Selektion für die Antigene beziehungsweise die Antikörper zu erhalten, welche auch mit BSP im Komplex mit Faktor H reagieren, wurde Faktor-H oder aus Knochen isoliertes oder gentechnisch hergestelltes BSP an Cyanogenbromid aktivierte Sepharose 4 B chemisch kovalent gebunden und danach soviel BSP beziehungsweise Faktor-H auf die Säule aufgetragen und gebunden, dass sämtliche Liganden in der Matrix mit dem Partner komplexiert waren. Dann wurde eine 1gG-Fraktion aus Serum von immunisierten Tieren auf diese Affinitätssäule aufgetragen und die Antikörperfraktion gewonnen, die spezifisch an das freie Epitop im BSP/Faktor-H-Komplex banden. Diese Versuche ergaben, dass insbesondere das 5er-Multimer vBSP5x des eBSP-Epitops in Impfbakterien in Kaninchen extrem hohe Antikörpertiter ergaben, wobei die resultierenden Antikörper humanes BSP im Komplex mit Komplementfaktor-H banden. Diese Antikörper werden im Tiermodel (Nacktratte mit menschlichen osteotrophen Tumorzellen), wie in der WO 2002/100899 beschrieben, auf ihre therapeutische Wirksamkeit getestet.

Für Immunisierung von Kaninchen wurden BSP-Impfbakterien mit dem Klon pIUSind-mBSP5x hergestellt. Das Plasmid pIUSind-mBSP5x wurde in *Listerien*-Zellen transformiert. Der Erfolg der Transformation wurde durch Präparation der Plasmid-DNA aus den transformierten Listerien Klonen und Restriktionsverdau der präparierten Plasmid-DNA verifiziert. Listeria pIUSind-mBSP5x Bakterien wurde in einer 5ml Kultur in einem geeigneten Kulturmedium angezogen. Die Verankerung der Tumorepitope erfolgte während der Anzucht von den Bakterien. Die BSP-Impfbakterien wurden durch Zentrifugation geerntet und durch Zugabe von Formaldehyd (1% Endkonzentration) über 24h bei Raumtemperatur inaktiviert. Die Qualität der BSP-Impfbakterien wurde durch Charakterisierung der inaktivierten Impfbakterien im Durchflusszytometer sichergestellt. Messungen der inaktivierten BSP-Impfbakterien im FACS ergaben, dass die Inaktivierung der Impfbakterien keine negativen Auswirkungen auf die Verankerung der BSP-Tumorepitope auf den Listerien zeigte. Bei der DSMZ hinterlegt sind die Organismen Listeria pIUSind-mBSP5x und Listeria pIEx-A-vBSP3 (DSM 18305, DSM 18306) als Vakzinbeispiele.

Der Shuttlevektor wurde in abgeschwächt pathogenen Gram-positiven Listeria-Bakterien transformiert. Bei *L.monocytogenes* ist nämlich der Weg in die Zelle von Mensch und Tier gut definiert. Für die volle Pathogenität der Listerien sind Faktoren nötig wie PrfA (positive regulator of virulence), ActA (actin nucleating protein), PIcA (phosphatidylinositol-specific phospholipase), PIcB (phosphatidyl-choline-specific phospholipase), Hly (listeriolysin), Mpl (metalloprotease), die für eine Abschwächung der Pathogenität gezielt ausgeschaltet werden können. Die Spezifität zwischen Pathogen und Wirtszelle wird unter anderem vermittelt durch die Internaline inIA und inIB. *L.monocytogenes* kann Endothelzellen, Epithelzellen, Fibroblasten und Hepatozyten infizieren, ferner neutrophile Granulocyten, Makrophagen, Lymphozyten und andere Zellen des weißen Blutbildes. Nach der Adhäsion an der Zelloberfläche wird *L.monocytogenes* durch Endozytose in die Zellen eingeschleust. Dann zerstört der Keim mithilfe von Listeriolysin (Hly) die Endosomenmembran, setzt sich im Zytosol der Wirtszelle frei, vermehrt sich dort unter der Produktion des klonierten Fragments und weiterer Proteine und befällt schließlich Nachbarzellen. In diesem Prozess wird das transgene hBSP-Peptid beziehungsweise die klonierte antigene Determinante von den Wirtszellen posttranslational modifiziert (O- und N-glykosyliert, sulfatiert, phosphoryliert, etc.) sezerniert beziehungsweise auf die Listeria-Bakterien gekoppelt. Es kann so eine Autoimmunreaktion gegen BSP und insbesondere gegen bestimmte Fragmente beziehungsweise Epitope des humanen BSP, einschließlich eventueller posttranslationaler Modifikationen, hervorgerufen werden. Wurden die Trägerbakterien in einem künstlichen Medium angezüchtet und vor einer Immunisierung inaktiviert, beispielsweise durch Behandlung mit Formalin oder Zusatz eines Antibiotikums, so lag die vom Trägerbakterium exprimierte antigene Determinante auf der Oberfläche des Trägerbakteriums vor. Das rekombinante Immuntag besitzt dann keine posttranslationalen Modifikationen.

### Zusammenfassung

Die Erfindung stellt somit eine therapeutische Zusammensetzung bereit zur Erzeugung von Antikörpern gegen das humane Bonesialoprotein (hBSP), welche spezifisch Epitope auf humanem Bonesialoprotein von Tumorzellen binden. Die therapeutische Zusammensetzung wurde insbesondere optimiert auf die Induktion von Antikörper, welche tumorgenes hBSP im Serum auch im Komplex mit dem Komplementfaktor-H erkennen. Die Erfindung umfasst weiterhin Mikroorganismen mit einem Vektorsystem für die Induktion einer zielgerichteten somatischen Transgenität in einem Wirt, insbesondere auch in einem menschlichen Patienten. Die erfindungsgemäße Zusammensetzung erstreckt sich insbesondere auf ein Vakzin zur Induktion von Antikörpern gegen Knochenproteine, die von Zellen osteotropher Tumor produziert und für eine Aussiedelung im Knochen benötigt werden. Es wird indirekt ausgenutzt, dass proliferierende Tumorzellen diese ursprünglich stark glykosylierten Proteine nicht mehr richtig posttranslational prozessieren können, so dass körpereigene Antikörper gegen die tumorgenen Proteine, welche an sich gleichfalls körpereigen sind, hergestellt werden können. Mit dem oben genanten Verfahren, Vakzinen und Wirkstoffen wurde weiterhin ein Verfahren zur Schutzimpfung und zur Behandlung osteotropher Tumoren bereitgestellt.

## Patentansprüche

1. Therapeutische Zusammensetzung zur Behandlung und Prophylaxe von Knochentumoren und Metastasen, die bevorzugt in Knochengeweben ansiedeln, enthaltend als Wirkstoff abgetötete oder schwach pathogene Mikroorganismen, die ein Gen für antigene Fragmente des Bonesialoproteins enthalten und ein oder mehrere Bonesialoprotein-Antigene exprimieren, welche sich in mindestens einem Strukturmerkmal von endogenem Bonesialoprotein normaler Osteoblasten unterscheidet, so dass bei einer Verabreichung eine Immunreaktion gegen das veränderte Bonesialoprotein erzeugt wird.

2. Therapeutische Zusammensetzung nach Anspruch 1, wobei die exprimierten Bonesialoprotein-Antigene Strukturmerkmale aufweisen, die spezifisch sind für eine Bonesialoprotein-Isoform, welche von osteotrophen Zellen eines Primärtumors exprimiert wird.

3. Therapeutische Zusammensetzung nach Anspruch 1 oder 2, wobei der Mikroorganismus ausgewählt ist aus Bakterien, Viren und Einzeller.

4. Therapeutische Zusammensetzung nach einem der Ansprüche 1 bis 3, wobei der Mikroorganismus ein Gram-positives Bakterium ist.

5. Therapeutische Zusammensetzung nach Anspruch 4, wobei das Bakterium Listeria ist, insbesondere Listeria monocytogenes.

6. Therapeutische Zusammensetzung nach Anspruch 3, wobei der Mikroorganismus ausgewählt ist aus den Spezies Aeromonas, Bartonella, Bruceila, Bacillen, Bacillus subtilis, Lactobacillen, Pseudomonaden, Staphylokokken, Yersinia Campylobacter, Clostridia, Enterobacteriaceae, Legionella, Mycobacterium, Renibacterium, Rhodococcus, Escherichia, Shigella, Salmonella, und Bakterien, die in einem eukaryonten Wirtsorganismus lebensfähig sind.

7. Therapeutische Zusammensetzung nach einem der Ansprüche 1 bis 5, wobei der Mikroorganismus eine antigene Determinante des Bonesialoproteins auf der Oberfläche verankert trägt.

8. Therapeutische Zusammensetzung nach Anspruch 7, wobei die antigene Determinante des Bonesialoproteins typisch ist für tumorgenes Bonesialoprotein.

9. Therapeutische Zusammensetzung nach Anspruch 7 oder 8, wobei die antigene Determinante unterglykosyliertes Bonesialoprotein-Antigen ist.

10. Therapeutische Zusammensetzung nach einem der Ansprüche 1 bis 9, wobei das Bonesialoprotein-Antigen ein oder mehrere Kopien folgender Aminosäuresequenzen enthält:
| | |
|---|---|
| YTGLAAIQLPKKAGD | SEQ ID NO. 5 |
| TGLAA | SEQ ID NO. 3 |
| YTGLAA | SEQ ID NO. 4 |
| YESENGEPRGDNYRAYED | SEQ ID NO 6 |
| LKRFPVQGG | SEQ ID NO: 7 |
| EDATPGTGYTGLAAIQLPKKAG | SEQ ID NO: 10 |

11. Therapeutische Zusammensetzung nach einem der Ansprüche 1 bis 10, umfassend als Wirkstoff ein isoliertes Fremdprotein mit einer antigenen Determinante , die typisch ist für tumorgenes Bonesialoprotein .

12. Therapeutische Zusammensetzung nach Anspruch 11, wobei der Wirkstoff die antigene Determinante des Bonesialoproteins in mindestens zwei Kopien oder mehrfach enthält.

13. Therapeutische Zusammensetzung nach einem der Ansprüche 1 bis 12, wobei das Bonesialoprotein-Antigen ein Epitop enthält, das im Komplex von Komplementfaktor-H und Bonesialoprotein für die Bindung eines Antikörpers frei ist.

14. Therapeutische Zusammensetzung nach einem der Ansprüche 1 bis 13, wobei die antigenen Determinanten auf peptidischen Molekülen oder Trägerproteinen mehrfach vorhanden sind.

15. Therapeutische Zusammensetzung nach einem der Ansprüche 1 bis 14, wobei die peptidischen Moleküle mit beta-Alanin gekoppelt sind.

16. Therapeutische Zusammensetzung nach einem der Ansprüche 1 bis 15, welche als Vakzin formuliert ist.

17. Therapeutische Zusammensetzung nach einem der Ansprüche 1 bis 16, formuliert für die Erzeugung einer Immunreaktion gegen den Tumor und dessen streuenden Tumorenzellen.

18. Therapeutische Zusammensetzung nach einem der vorhergehenden Ansprüche zur Behandlung von Tumoren aus der Gruppe mit Prostata-, Mamma-, Lungen-, Nieren- und Schilddrüsentumoren, Tumorerkrankungen des Blutsystems, des Lymphsystems, des Herz-Kreislauf-Systems, des Nervensystems, des Respirationstraktes, des Verdauungstraktes, des endokrinen Systems, der Haut einschließlich Anhangsgebilde, des Bewegungsapparates und des Urogenitaltraktes, einschließlich der Niere.

## Claims

1. A therapeutic composition for the treatment and prophylaxis of bone tumors and metastases which preferentially settle in bone tissues, comprising as active ingredient deadened or weakly pathogenic microorganisms comprising a gene for antigenic fragments of bonesialoprotein and which express one or more bonesialoprotein antigens that differ from endogenous bonesialoprotein of normal osteoblasts in at least one structural feature so that an immune reaction against the modified bonesialoprotein is induced after administration.

2. A therapeutic composition according to claim 1, wherein the expressed bonesialoprotein antigens displays structural features which are specific for a bonesialoprotein isoform expressed by osteotrophic cells of a primary tumor.

3. A therapeutic composition according to claim 1 or 2, wherein the microorganism is chosen from bacteria, viruses and monads.

4. A therapeutic composition according to any one of claims 1 to 3, wherein the microorganism is a gram-positive bacterium.

5. A therapeutic composition according to claim 4, wherein the bacterium is Listeria, particularly Listeria monocytogenes.

6. A therapeutic composition according to claim 3, wherein the microorganism is chosen from the species Aeromonas, Bartonella, Brucella, Bacilli, Bacillus subtilis, Lactobacilli, Pseudomonades, Staphylococci, Yersinia, Campylobacter, Clostridia, Enterobacteriaceae, Legionella, Mycobacterium, Renibacterium, Rhodococcus, Escherichia, Shigella, Salmonella, and bacteria, which are viable in a eucaryotic host organism.

7. A therapeutic composition according to any one of claims 1 to 5, wherein the microorganism carries an antigenic determinant of the bonesialoprotein anchored to its surface

8. A therapeutic composition according to claim 7, wherein the antigenic determinant of the bonesialoprotein is typical for tumorgenic bonesialoprotein.

9. A therapeutic composition according to claim 7 or claim 8, wherein the antigenic determinant is underglycosylated bonesialoprotein antigen.

10. A therapeutic composition according to any one of claims 1 to 9, wherein the bonesialoprotein antigen comprises one or more copies of the following amino acid sequences:
| | |
|---|---|
| YTGLAAIQLPKKAGD | SEQ. ID NO. 5 |
| TGLAA | SEQ. ID NO. 3 |
| YTGLAA | SEQ. ID NO. 4 |
| YESENGEPRGDNYRAYED | SEQ. ID NO. 6 |
| LKRFPVQGG | SEQ. ID NO. 7 |
| EDATPGTGYTGLAAIQLPKKAG | SEQ. ID NO. 10 |

11. A therapeutic composition according to any of claims 1 to 10 including as an active ingredient an isolated foreign protein having an antigenic determinant, which is characteristic for tumorgenic bonesialoprotein.

12. A therapeutic composition according to claim 11, wherein the active ingredient comprises the antigenic determinant of bonesialoprotein in at least two or more copies.

13. A therapeutic composition according to any of claims 1 to 12, wherein the bonesialoprotein antigen comprises an epitope which is freely accessible complex of complement factor H and bonesialoprotein for binding of an antibody.

14. A therapeutic composition according to any one of claims 1 to 13, wherein the antigenic determinants are multiply present on peptidic molecules or carrier proteins.

15. A therapeutic composition according to any one of claims 1 to 14, wherein the peptidic molecules are coupled to beta-alanine.

16. A therapeutic composition according to any one of claims 1 to 15, formulated as a vaccine.

17. A therapeutic composition according to any one of claims 1 to 16, formulated for inducing an immune reaction against a tumor and its disseminating tumor cells.

18. A therapeutic composition according to any preceding claim for treatment of tumors from the group with prostate, breast, lung, kidney and thyroid tumors, and tumors of the circulatory system, the lymphoid system, the cardiovascular system, the neurological system, the respiratory tract, the digestive tract, the endocrinal system, the skin including adnexa, the muscoskeletal system and the urogenital tract, including the kidneys.

## Revendications

1. Composition thérapeutique pour le traitement et la prévention de tumeurs osseuses et de métastases qui s'implantent préférentiellement dans des tissus osseux, comprenant comme agent actif des microorganismes tués ou légèrement pathogènes qui comprennent un gène pour des fragments antigènes de sialoprotéine osseuse et qui expriment un ou plusieurs antigènes de sialoprotéine osseuse différents de sialoprotéine osseuse endogène issue d'ostéoblastes normaux par au moins une caractéristique structurelle, de façon qu'une réaction immunologique contre la sialoprotéine osseuse modifiée est créée après administration.

2. Composition thérapeutique selon la revendication 1 dans laquelle les antigènes de sialoprotéine osseuse exprimés ont des caractéristiques structurelles qui sont spécifiques pour une isoforme de sialoprotéine osseuse qui est exprimée par des cellules ostéotrophes d'une tumeur primaire.

3. Composition thérapeutique selon la revendication 1 ou 2 dans laquelle le microorganisme est choisi parmi bactéries, virus et organismes unicellulaires.

4. Composition thérapeutique selon l'une des revendications 1 à 3 dans laquelle le microorganisme est une bactérie à Gram positif.

5. Composition thérapeutique selon la revendication 4 dans laquelle la bactérie est listeria, en particulier listeria monocytogenes.

6. Composition thérapeutique selon la revendication 3 dans laquelle le microorganisme est choisi parmi les espèces Aeromonas, Bartonella, Brucella, Bacillus, Bacillus subtilis, Lactobacillus, Pseudomonades, Staphylococcus, Yersinia, Campylobacter, Clostridia, Enterobacteriaceae, Legionella, Mycobacterium, Renibacterium, Rhodococcus, Escherichia, Shigella, Salmonella, et des bactéries qui peuvent survivre dans un organisme hôte eucaryote.

7. Composition thérapeutique selon l'une des revendications 1 à 5 dans laquelle le microorganisme porte un déterminant antigène de la sialoprotéine osseuse ancré sur sa surface.

8. Composition thérapeutique selon la revendication 7 dans laquelle le déterminant antigène de la sialoprotéine osseuse est caractéristique pour de la sialoprotéine osseuse génératrice de tumeurs.

9. Composition thérapeutique selon la revendication 7 ou 8 dans laquelle le déterminant antigène est un antigène de sialoprotéine osseuse sous-glycosylé.

10. Composition thérapeutique selon l'une des revendications 1 à 9 dans laquelle l'antigène de sialoprotéine osseuse comprend une ou plusieurs copies des séquences d'acides aminés suivantes :
| | |
|---|---|
| YTGLAAIQLPKKAGD | SEQ. ID NO. 5 |
| TGLAA | SEQ. ID NO. 3 |
| YTGLAA | SEQ. ID NO. 4 |
| YESENGEPRGDNYRAYED | SEQ. ID NO. 6 |
| LKRFPVQGG | SEQ. ID NO. 7 |
| EDATPGTGYTGLAAIQLPKKAG | SEQ. ID NO. 10 |

11. Composition thérapeutique selon l'une des revendications 1 à 10 comprenant comme agent actif une protéine étrangère isolée avec un déterminant antigène qui est typique pour de la sialoprotéine osseuse génératrice de tumeurs.

12. Composition thérapeutique selon la revendication 11 dans laquelle l'agent actif comprend le déterminant antigène de la sialoprotéine osseuse en au moins deux ou plusieurs copies.

13. Composition thérapeutique selon l'une des revendications 1 à 12 dans laquelle l'antigène de sialoprotéine osseuse comprend un épitope qui est libre pour la liaison d'un anticorps s'il est complexé dans un facteur complément H et à de la sialoprotéine osseuse.

14. Composition thérapeutique selon l'une des revendications 1 à 13 dans laquelle les déterminants antigènes sont présents plusieurs fois sur des molécules peptidiques ou des protéines vecteurs.

15. Composition thérapeutique selon l'une des revendications 1 à 14 dans lesquelles les molécules peptidiques sont liées à de la béta-alanine.

16. Composition thérapeutique selon l'une des revendications 1 à 15 formulée comme vaccin.

17. Composition thérapeutique selon l'une des revendications 1 à 16 formulée pour la création d'une réaction immunologique contre la tumeur et ses cellules tumorales disséminantes.

18. Composition thérapeutique selon l'une des revendications précédentes pour le traitement de tumeurs du groupe avec des tumeurs de la prostate, du sein, du poumon, du rein et de la thyroïde, des maladies tumorales du système circulaire, du système lymphoïde, du système cardio-vasculaire, du système nerveux, des voies respiratoires, de l'appareil digestif, du système endocrinien, de la peau y compris ses annexes, de l'appareil locomoteur et du système urogénital, y compris le rein.


### SEQUENCE LISTING

<110> JOCHEM. Ralf
   <120> Therapeutische zusammensetzung zur Vorbeugung und Bekämpfung von Knochenmetastasen
<130> GP100039PC00
<150> DE102005024836.5
   <151> 2005-05-31
<150> DE102006004612.9
   <151> 2006-02-01
<160> 16
<170> PatentIn version 3.3
<210> 1
   <211> 17
   <212> PRT
   <213> Homo sapiens
<220>
   <221> misc_feature
   <222> (1)..(1)
   <223> xaa can be any naturally occurring amino acid
<400> 1
   Xaa Tyr Thr Gly Leu Ala Ala Ile Gln Leu Pro Lys Lys Ala Gly Asp
   1               5                   10                  15
   Glx
<210> 2
   <211> 299
   <212> PRT
   <213> Homo sapiens
<220>
   <221> misc_feature
   <222> (1)..(1)
   <223> xaa can be any naturally occurring amino acid
<400> 2
   Xaa Phe Ser Met Lys Asn Leu His Arg Arg Val Lys Ile Glu Asp Ser
   1               5                   10                  15
   Glu Glu Asn Gly Val Phe Lys Tyr Arg Pro Arg Tyr Tyr Leu Tyr Lys
               20                  25                  30
   His Ala Tyr Phe Tyr Pro His Leu Lys Arg Phe Pro Val Gln Gly Ser
           35                  40                  45
   Ser Asp Ser Ser Glu Glu Asn Gly Asp Asp Ser Ser Glu Glu Glu Glu
       50                  55                  60
   Glu Glu Glu Glu Thr Ser Asn Glu Gly Glu Asn Asn Glu Glu Ser Asn
   65                  70                  75                  80
   Glu Asp Glu Asp Ser Glu Ala Glu Asn Thr Thr Leu Ser Ala Thr Thr
                   85                  90                  95
   Leu Gly Tyr Gly Glu Asp Ala Thr Pro Gly Thr Gly Tyr Thr Gly Leu
               100                 105                 110
   Ala Ala Ile Gln Leu Pro Lys Lys Ala Gly Asp Ile Thr Asn Lys Ala
           115                 120                 125
   Thr Lys Glu Lys Glu Ser Asp Glu Glu Glu Glu Glu Glu Glu Glu Gly
       130                 135                 140
   Asn Glu Asn Glu Glu Ser Glu Ala Glu Val Asp Glu Asn Glu Gln Gly
   145                 150                 155                 160
   Ile Asn Gly Thr Ser Thr Asn Ser Thr Glu Ala Glu Asn Gly Asn Gly
                   165                 170                 175
   Ser Ser Gly Val Asp Asn Gly Glu Glu Gly Glu Glu Glu Ser Val Thr
               180                 185                 190
   Gly Ala Asn Ala Glu Gly Thr Thr Glu Thr Gly Gly Gln Gly Lys Gly
           195                 200                 205
   Thr Ser Lys Thr Thr Thr Ser Pro Asn Gly Gly Phe Glu Pro Thr Thr
       210                 215                 220
   Pro Pro Gln Val Tyr Arg Thr Thr Ser Pro Pro Phe Gly Lys Thr Thr
   225                 230                 235                 240
   Thr Val Glu Tyr Glu Gly Glu Tyr Glu Tyr Thr Tyr Asp Asn Gly Tyr
                   245                 250                 255
   Glu Ile Tyr Glu Ser Glu Asn Gly Glu Pro Arg Gly Asp Asn Tyr Arg
               260                 265                 270
   Ala Tyr Glu Gly Glu Tyr Ser Tyr Phe Lys Gly Gln Gly Tyr Asp Gly
           275                 280                 285
   Tyr Asp Gly Gln Asn Tyr Tyr His His Gln Glx
       290                 295
<210> 3
   <211> 5
   <212> PRT
   <213> Homo sapiens
<400> 3
   Thr Gly Leu Ala Ala
   1 5
<210> 4
   <211> 6
   <212> PRT
   <213> Homo sapiens
<400> 4
   Tyr Thr Gly Leu Ala Ala
   1 5
<210> 5
   <211> 15
   <212> PRT
   <213> Homo sapiens
<400> 5
   Tyr Thr Gly Leu Ala Ala Ile Gln Leu Pro Lys Lys Ala Gly Asp
   1 5 10 15
<210> 6
   <211> 18
   <212> PRT
   <213> Homo sapiens
<400> 6
   Tyr Glu Ser Glu Asn Gly Glu Pro Arg Gly Asp Asn Tyr Arg Ala Tyr
   1 5 10 15
   Glu Asp
<210> 7
   <211> 9
   <212> PRT
   <213> Homo sapiens
<400> 7
   Leu Lys Arg Phe Pro Val Gln Gly Gly
   1 5
<210> 8
   <211> 7
   <212> PRT
   <213> Homo sapiens
<400> 8
   Ser Gly Gly Gly Gly Ser Ala
   1 5
<210> 9
   <211> 7
   <212> PRT
   <213> Homo sapiens
<400> 9
   Asp Ala Thr Pro Gly Thr Gly
   1 5
<210> 10
   <211> 22
   <212> PRT
   <213> Homo sapiens
<400> 10
   Glu Asp Ala Thr Pro Gly Thr Gly Tyr Thr Gly Leu Ala Ala Ile Gln
   1 5 10 15
   Leu Pro Lys Lys Ala Gly
   20
<210> 11
   <211> 12
   <212> PRT
   <213> Homo sapiens
<400> 11
   Leu Gly Tyr Gly Glu Asp Ala Thr Pro Gly Thr Gly
   1 5 10
<210> 12
   <211> 12
   <212> PRT
   <213> Homo sapiens
<400> 12
   Glu Thr Gly Gly Gln Gly Lys Gly Thr Ser Lys Thr
   1 5 10
<210> 13
   <211> 12
   <212> PRT
   <213> Homo sapiens
<400> 13
   Phe Lys Gly Gln Gly Tyr Asp Gly Tyr Asp Gly Gln
   1 5 10
<210> 14
   <211> 15
   <212> PRT
   <213> Homo sapiens
<400> 14
   Ile Gln Leu Pro Lys Lys Ala Gly Asp Ile Thr Asn Lys Ala Thr
   1 5 10 15
<210> 15
   <211> 17
   <212> PRT
   <213> Homo sapiens
<400> 15
   Gly Asp Ile Thr Asn Lys Ala Thr Lys Glu Lys Glu Lys Glu Ser Asp
   1 5 10 15
   Glu
<210> 16
   <211> 38
   <212> PRT
   <213> Homo sapiens
<400> 16
   Ser Glu Asn Gly Glu Pro Arg Gly Asp Asn Tyr Arg Ala Tyr Glu Asp
   1 5 10 15
   Glu Tyr Ser Tyr Phe Lys Gly Gln Gly Tyr Asp Gly Tyr Asp Gly Gln
   20 25 30
   Asn Tyr Tyr His His Gln
   35